# EUROPÄISCHE PATENTSCHRIFT

(11) **EP 1 217 955 B1**
(45) Veröffentlichungstag und Bekanntmachung des Hinweises auf die Patenterteilung: **14.05.2003**
(21) Anmeldenummer: 00974310.5
(22) Anmeldetag: 05.09.2000
(51) Int. Cl.: A61B 17/04, A61B 17/00

(54) **VORRICHTUNG ZUM HINDURCHFÜHREN WENIGSTENS ZWEIER NÄHFÄDEN DURCH EINE WANDUNG, INSBESONDERE EINER ARTERIE EINES INDIVIDUUMS, NAHE DES RANDBEREICHES EINER DARIN VORHANDENEN ÖFFNUNG**
DEVICE FOR GUIDING AT LEAST TWO SUTURES THROUGH A WALL, IN PARTICULAR AN ARTERIAL WALL OF AN INDIVIDUAL, IN CLOSE PROXIMITY TO THE EDGE OF AN OPENING IN SAID WALL
DISPOSITIF DE GUIDAGE D'AU MOINS DEUX SUTURES A TRAVERS UNE PAROI, NOTAMMENT UNE PAROI ARTERIELLE D'UN SUJET, A PROXIMITE DU BORD D'UNE OUVERTURE PRATIQUEE DANS LA DITE PAROI

(30) Priorität: 08.09.1999 DE 19942951
(43) Veröffentlichungstag der Anmeldung: 03.07.2002
(73) Patentinhaber: Medi-Globe GmbH, 83101 Rohrdorf-Achenmühle (DE)
(72) Erfinder: ISCHINGER, Thomas, Prof. Dr. med., 81925 München (DE); BOEHLKE, Raimar, 83064 Raubling (DE)
(74) Vertreter: Nätebusch, Roderich
(86) Internationale Anmeldenummer: DE0003118
(87) Internationale Veröffentlichungsnummer: WO01017434

(56) Entgegenhaltungen:
- US-A- 5 304 184
- US-A- 5 417 699
- US-A- 5 527 322
- US-A- 5 860 990

## Beschreibung

Die Erfindung bezieht sich auf eine Vorrichtung zum Hindurchführen wenigstens zweier Nähfäden durch eine Wandung einer Hülle, eines Ballons oder einer Fläche, insbesondere einer Arterie eines Individuums nahe des Randbereiches eines darin vorhandenen, gegebenenfalls durch Ein- und/oder Freischneiden gebildeten Öffnung und zum Zurückziehen der durch den betreffenden Randbereich hindurchgeführten Nähfädenenden aus der genannten Öffnung, mit einer stabförmigen Fadenführungseinrichtung, in der die an Nadeln befestigten Nähfäden in Führungs- bzw. Aufnahmeöffnungen derart geführt sind, dass sie mittels der betreffenden Fadenführungseinrichtung nahe des Randbereiches der betreffenden Öffnung durch die genannte Wandung hindurchführbar und aus der genannten öffnung wieder derart zurückziehbar sind, dass durch Zusammenziehen und gegebenenfalls Verknoten der Nähfädenenden außerhalb der betreffenden Öffnung diese verschließbar ist.

Eine Vorrichtung der vorstehend genannten Art kommt hauptsächlich in solchen Fällen zum Einsatz, in denen in der Wandung einer Hülle, eines Ballons oder einer Fläche eine Öffnung vorhanden ist, die geschlossen werden soll und für deren Verschließen ein Zugang von beiden Seiten der betreffenden Wandung aus nicht möglich ist. Dies ist insbesondere dann der Fall, wenn die die zu verschließende Öffnung enthaltende Wandung zu einer Arterie eines Individuums gehört. In diesen Fällen ist es lediglich möglich, von der Außenseite her Zugang zu der jeweils zu verschließenden Öffnung zu erhalten.

Es ist bereits eine Nähvorrichtung der eingangs genannten Art bekannt (WO 94/08516), bei der bis zu vier mit Nähfäden verbundene Nadeln mittels einer Schiebeeinrichtung durch eine Wandung eines Blutgefäßes nahe des Randbereiches einer darin befindlichen Öffnung hindurchgeführt und von einer innerhalb des betreffenden Blutgefäßes im Bereich der genannten Öffnung befindlichen Aufnahmeeinrichtung aufgenommen werden können. Diese Aufnahmeeinrichtung ist durch eine fangkorbartige Nadelaufnahmeeinrichtung gebildet, die zunächst im zusammengelegten Zustand durch die betreffende öffnung hindurchgeführt und sodann in der betreffenden Öffnung aufgeweitet wird, um daraufhin am Randbereich der betreffenden Öffnung innerhalb des Blutgefäßes anzuliegen. Die durch den Randbereich der erwähnten Öffnung hindurchgeführten Nadeln sind von dieser fangkorbartigen Nadelaufnahmeeinrichtung aufnehmbar, die anschließend um ihre Längsachse verdreht wird, um die betreffenden Nadeln festzuhalten. Danach wird die betreffende fangkorbartige Nadelaufnahmeeinrichtung mit den in ihr enthaltenen Nadeln zusammengelegt, um durch die erwähnte Öffnung vollständig herausgezogen zu werden. Bei diesem Vorgang werden die mit den Nadelenden verbundenen Nähfäden aus Vorratsmagazinen herausgezogen, durch die Einstichstellen der erwähnten Nadeln nahe des Randbereiches der erwähnten Öffnung hindurchgezogen und aus der betreffenden Öffnung wieder herausgezogen. Außerhalb der erwähnten Öffnung können die Nähfädenenden dann zusammengezogen und gegebenenfalls verknotet werden, um die betreffende Öffnung zu verschließen.

Obwohl die vorstehend betrachtete bekannte Vorrichtung vom Prinzip her einen relativ einfachen Aufbau zeigt, können sich jedoch bei der Aufnahme und beim Festhalten der nahe des Randbereiches der jeweiligen Öffnung hinduchgeführten Nadeln in der fangkorbartigen Nadelaufnahmeeinrichtung Probleme ergeben, wenn eine oder mehrere Nadeln durch diese Nadelaufnahmeeinrichtung nicht festgehalten werden können. In solchen Fällen sind dann komplizierte Eingriffe erforderlich, um die in dem Blutgefäß enthaltenen Nadeln wieder zu entfernen.

Es ist ferner eine Vorrichtung zum Hindurchführen zweier Nähfäden durch eine Wandung eines Blutgefäßes nahe des Randbereiches einer darin gebildeten Öffnung bekannt (WO 94/13211), die einen in die betreffende Öffnung einzuführenden Nadelträger und eine außerhalb der betreffenden Öffnung befindliche Nadelaufnahmeeinrichtung enthält. Der Nadelträger ist mit Nähfäden ausgestattet, deren Enden mit Nadeln verbunden sind. Im Betrieb wird der erwähnte Nadelträger zunächst vollständig durch die erwähnte Öffnung in das zu verschließende Blutgefäß eingeführt, und anschließend werden die Nadeln durch den Randbereich der betreffenden Öffnung von innen nach außen hindurchgeführt. Dabei verbleiben die restlichen Nadelträgerteile zunächst noch in dem Blutgefäß. Anschließend werden diese Nadelträgerteile aus dem Blutgefäß durch die erwähnte Öffnung herausgezogen, so dass in dem betreffenden Blutgefäß lediglich der mit den bereits herausgeführten Nadeln verbundene Nähfaden verbleibt. Durch weiteres Zurückziehen der Nadeln wird der betreffende Nähfaden schließlich im Innern des betreffenden Blutgefäßes gespannt, so dass danach die Nähfädenenden verknotet werden können. Dadurch ist dann die betreffende Öffnung verschlossen.

Die gerade betrachtete bekannte Vorrichtung gestattet zwar vom Prinzip her Nähfäden durch die Wandung eines Blutgefäßes nahe des Randbereiches einer darin vorhandenen öffnung hindurchzuführen; dabei ist jedoch auch hier die Sicherheit im Zusammenhang mit der Aufnahme der Nadelenden in der Nadelaufnahmeeinrichtung zumindest kritisch. Wird die eine oder andere Nadel nämlich nicht sicher von der Nadelaufnahmeeinrichtung aufgenommen, so sind auch in diesem Fall zusätzliche Eingriffe zur Vermeidung von Komplikationen notwendig. Aus Dimensionierungsgründen muß hier der Zugang zur Arterie aufgeweitet werden. Die betreffende bekannte Vorrichtung ist somit nicht minimalinvasiv.

Es ist außerdem eine Vorrichtung zum Hindurchführen wenigstens eines Nähfadens durch die Wandung eines Blutgefäßes eines Individuums nahe des Randbereiches einer darin vorhandenen Öffnung bekannt (WO 95/13021). Diese bekannte Vorrichtung weist eine stabförmige Fadenführungseinrichtung auf, an deren Spitze ein Nasenteil mit einer Nadelumlenkbahn vorgesehen ist, welches über einen Bereich verminderten Querschnitts mit der stabförmigen Fadenführungseinrichtung verbunden ist. In dieser stabförmigen Fadenführungseinrichtung gibt es eine Nadelzuführöffnung, die zur Eintrittsseite der Nadelumlenkbahn im Nasenteil ausgerichtet ist. Außerdem gibt es in der stabförmigen Fadenführungseinrichtung eine zweite Nadelführungsöffnung, die zu der Austrittsseite der Nadelumlenkbahn im Nasenteil ausgerichtet ist. Aufgrund dieses Aufbaus funktioniert die gerade betrachtete bekannte Vorrichtung wie folgt. Zunächst wird die gesamte Vorrichtung in die zu verschließende Öffnung eines Blutgefäßes eines Individuums soweit eingeführt, dass die Wandung der betreffenden öffnung an dem erwähnten Bereich verminderten Querschnitts anliegt, über den das Nasenteil mit der stabförmigen Fadenführungseinrichtung verbunden ist. Sodann wird eine mit einem Nähfaden verbundene Nadel durch die Nadelzuführöffnung der stabförmigen Fadenführungseinrichtung nach vorn zu dem Nasenteil hin bewegt, wobei die betreffende Nadel dabei die Wand des Blutgefäßes nahe des Randbereiches der erwähnten Öffnung durchsticht und anschließend in der Umlenkbahn des Nasenteiles derart umgelenkt wird, dass es sodann den Randbereich der betreffenden Gefäßwand von innen nach außen durchsticht. Daraufhin wird die betreffende Nadel durch die erwähnte weitere Nadelführungsöffnung der stabförmigen Fadenführungseinrichtung wieder zurückgeführt, so dass damit der Randbereich der erwähnten Öffnung an zwei diametral gegenüberliegenden Stellen nunmehr von einem Faden durchzogen ist. Dieser Faden muß dann anschließend aus der Umlenkbahn über eine mit dieser verbundene Fadenfreigabe-Schlitzanordnung herausgeführt werden, so dass daraufhin die gesamte Vorrichtung aus der Öffnung des erwähnten Blutgefäßes herausgezogen werden kann.

Die vorstehend betrachtete bekannte Vorrichtung gestattet zwar vom Prinzip her, wenigstens einen Nähfaden durch die Wand eines Blutgefäßes nahe des Randbereiches einer darin vorhandenen Öffnung an zwei diametral gegenüberliegenden Stellen hindurchzuführen; die erwähnte Nadelumlenkkonstruktion bereitet jedoch in der Praxis zuweilen Probleme, da infolge der relativ starken Krümmung der in dem erwähnten Nasenteil vorhandenen Umlenkbahn nur flexible Nadeln oder kleine Nadeln verwendbar sind, was indessen Probleme beim Hindurchführen solcher Nadeln durch Gefäßwände verursachen kann.

Es ist schließlich auch schon eine Nähvorrichtung zum Hindurchführen der Enden eines Nähfadens durch die Wand eines Blutgefäßes eines Individuums nahe des Randbereiches einer darin vorhandenen Öffnung bekannt (US 5.860.990). Bei dieser bekannten Vorrichtung wird ein Nähfadenvorrat mit schlaufenförmigen Fadenenden mittels einer stabförmigen Fadenzuführeinrichtung durch die betreffende Öffnung in das Blutgefäß eingeführt. Außerhalb der betreffenden stabförmigen Fadenzuführeinrichtung sind an zwei diametral gegenüberliegenden Stellen nadeiförmige Fadenaufnehmer vorgesehen, die nach Durchstechen der Gefäßwand nahe des Randbereiches der betreffenden Öffnung die schlaufenförmig ausgebildeten Fadenenden aufnehmen und dann nach außen aus dem Blutgefäß herausziehen sollen.

Obwohl die zuletzt betrachtete bekannte Vorrichtung vom Prinzip her schlaufenförmig ausgebildete Enden eines Nähfadens durch eine Blutgefäßwand nahe des Randbereiches einer darin vorhandenen Öffnung hindurchzuführen gestattet, zeigt indessen auch diese Konstruktion bei ihrem Einsatz Probleme mit der Aufnahme der erwähnten schlaufenförmigen Fadenenden. Eine sichere Aufnahme der betreffenden schlaufenförmigen Fadenenden ist nämlich nur dann gewährleistet, wenn die betreffenden Fadenenden von der stabförmigen Fadenzuführeinrichtung in einer solchen definierten Position festgehalten werden, in der die nadelförmigen Fadenaufnehmer diese schlaufenförmigen Fadenenden auch erfassen können. Dies ist in der Praxis zuweilen nur mit erheblichen Schwierigkeiten erreichbar.

Der Erfindung liegt demgemäß die Aufgabe zugrunde, einen Weg zu zeigen, wie bei einer Vorrichtung der eingangs genannten Art auf relativ einfache aber dennoch sichere Weise das Hindurchführen der wenigstens zwei vorgesehenen Nähfäden durch die Wandung einer Hülle, eines Ballons oder einer Fläche, insbesondere einer Arterie eines Individuums nahe des Randbereiches einer darin vorhandenen Öffnung erreicht wird.

Gelöst wird die vorstehend aufgezeigte Aufgabe bei einer Vorrichtung der eingangs genannten Art erfindungsgemäß dadurch, dass die Fadenführungseinrichtung in ihrer Längsrichtung ein hinteres Fadenzuführungsteil, ein vorderes Fadenaufnahmeteil und ein dazwischen liegendes mittleres Fadenfreigabe-/Fadenklemmteil aufweist und dass das genannte mittlere Fadenfreigabe-/Fadenklemmteil zumindest relativ zu dem vorderen Fadenaufnahmeteil verdrehbar ist und einen solchen Querschnitt aufweist, dass es in zumindest einer Drehstellung die Einführung der von dem hinteren Fadenzuführungsteil zugeführten Nähfäden in in dem vorderen Fadenaufnahmeteil freiliegende Aufnahmeöffnungen ermöglicht und in einer von der genannten Drehstellung verschiedenen Drehpositionen die in den betreffenden Aufnahmeöffnungen zusammen mit den Nadeln aufgenommenen Nähfäden für ein herausziehen der gesamten Fadenführungseinrichtung aus der genannten Öffnung heraus festzuhalten gestattet.

Die Erfindung bringt den Vorteil mit sich, dass sie mit relativ geringem Aufwand ein sicheres Hindurchführen wenigstens zweier Nähfäden durch die Wandung einer Hülle, eines Ballons oder einer Fläche, insbesondere einer Arterie eines Individuums nahe des Randbereiches einer darin vorhandenen Öffnung und das Zurückziehen der nahe des erwähnten Randbereiches hindurchgeführten Nähfäden aus der betreffenden Öffnung gewährleistet. Die betreffende Öffnung kann für einen wirksamen Einsatz der Vorrichtung gemäß der Erfindung für deren Einführung gegebenenfalls freigeschnitten sein. Dabei nutzt die vorliegende Erfindung ein relativ einfaches Konstruktionsprinzip, gemäß dem lediglich das mittlere Fadenfreigabe-/Fadenklemmteil relativ zu den anderen Vorrichtungsteilen verdrehbar zu sein braucht, um in einer Drehstellung, einer Fadenfreigabestellung das Hindurchführen der Nähfäden nahe des Randbereiches der erwähnten Öffnung zu ermöglichen, und in einer anderen Drehposition, einer Fadenklemmposition, die mit den Nähfäden in dem vorderen Fadenaufnahmeteil aufgenommenen Nadeln so festzuklemmen, dass die gesamte Vorrichtung aus der betreffenden Öffnung herausgezogen werden kann. Dabei werden die Nähfäden durch die erwähnte Wandung nahe des Randbereiches der genannten Öffnung nachgezogen, um anschließend außerhalb dieser Öffnung zusammengezogen und gegebenenfalls verknotet zu werden. Für diesen hier nicht weiter beschriebenen Vorgang kann dann ein an sich bekannter Knotenschieber eingesetzt werden. Als Nähfäden kommen chirurgische Nähfäden für den Fall in Betracht, dass die Vorrichtung gemäß der Erfindung eine Arterien- bzw. allgemein Blutgefäß-Verschließvorrichtung ist. Dadurch können dann Eversionsnähte hergestellt werden. Im Falle der Anwendung der vorliegenden Erfindung zum Verschließen einer Arterie ergibt sich der Vorteil eines minimalinvasiven Verschließens.

Zweckmäßige Weiterbildungen der Erfindung sind in den Unteransprüchen erfaßt.

Das hintere, das heißt das am proximalen Ende der Vorrichtung vorhandene Fadenzuführungsteil, das mittlere Fadenfreigabe-/Fadenklemmteil und das vordere, das heißt das am distalen Ende der Vorrichtung vorhandene Fadenaufnahmeteil weisen zweckmäßigerweise jeweils einen ovalförmigen Querschnitt auf. Dies ermöglicht eine optimale Arbeitsweise der betreffenden Vorrichtung, was insbesondere in dem Fall von Nutzen ist, dass die erwähnte Öffnung sich in einer Arterienwand eines Individuums, Mensch oder Tier, befindet. Diese Arterienwand kann sich dann vollständig an den ovalförmigen Querschnitt der betreffenden Vorrichtungsteile anlegen. Das mittlere Fadenfreigabe-/Fadenklemmteil dient in diesem Fall, wie noch ersichtlich werden wird, als Wundrandspanner in der zu verschließenden Arterienwandöffnung.

Dabei genügt es zuweilen, wenn das hintere Fadenzuführungsteil, das mittlere Fadenfreigabe-/Fadenklemmteil und das vordere Fadenaufnahmeteil der Fadenführungseinrichtung zumindest in ihren angrenzenden Bereichen jeweils denselben ovalförmigen Querschnitt aufweisen. Dies ermöglicht in vorteilhafter Weise ein problemloses Einführen und Herausführen der gesamten Vorrichtung in die jeweilige Öffnung bzw. aus dieser heraus, nahe deren Randbereich wenigstens zwei Nähfäden durch die die betreffende Öffnung enthaltende Wandung hindurchzuführen sind.

Von besonderem Vorteil ist es ferner, wenn der Querschnitt des mittleren Fadenfreigabe-/Fadenklemmteiles gegenüber den Querschnitten des hinteren Fadenzuführungsteiles und des vorderen Fadenaufnahmeteiles in der Dicke vermindert ist. Dadurch kann sich das betreffenden Fadenfreigabe-/Fadenklemmteil derart an den Rand der erwähnten Öffnung anlegen, nahe deren Randbereich durch die die betreffende Öffnung enthaltende Wandung wenigstens zwei Nähfäden hindurchzuführen sind, so dass die Durchsteckstellen für das Hindurchführen der betreffenden Nähfäden vom Rand der erwähnten Öffnung möglichst weit weg liegen. Dies ist für das Verschließen einer in einer Arterienwand vorhandenen Öffnung von ganz erheblichem Vorteil.

Von besonderem Vorteil ist es auch, wenn das hintere Fadenzuführungsteil, das mittlere Fadenfreigabe-/Fadenklemmteil und das vordere Fadenaufnahmeteil der Fadenführungseinrichtung alle relativ zueinander verdrehbar sind. Diese Verdrehbarkeit der einzelnen Vorrichtungsteile relativ zueinander ermöglicht eine sehr flexible Arbeitsweise, was insbesondere in den Fällen von Nutzen ist, dass mehr als zwei Nähfäden nahe des Randbereiches einer in der Wandung einer Hülle, eines Ballons oder einer Fläche, insbesondere einer Arterie eines Individuum vorhandenen Öffnung durch diese Wandung hindurchzuführen sind.

Eine besonders einfache Vorrichtungskonstruktion erreicht man dann, wenn das mittlere Fadenfreigabe-/Fadenklemmteil durch einen Bereich verminderten Querschnitts des hinteren Fadenzuführungsteiles gebildet ist. Dadurch kommt man in vorteilhafter Weise mit nur zwei relativ zueinander verdrehbaren Vorrichtungsteilen aus.

Bei dieser gerade betrachteten Vorrichtungskonstruktion kann der Bereich verminderten Querschnitts des hinteren Fadenzuführungsteiles zweckmäßigerweise durch ein gesondertes Teil gebildet sein, welches mit dem hinteren Fadenzuführungsteil verbunden ist. Dadurch ist die Herstellung der betreffenden Vorrichtungsteile relativ leicht möglich.

Vorzugsweise weist das mittlere Fadenfreigabe-/Fadenklemmteil eine der Dicke der Wandung einer Hülle, eines Ballons oder einer Fläche, insbesondere einer Arterie eines Individuums entsprechende Dicke auf. Dadurch lassen sich in optimaler Weise wenigstens zwei Nähfäden nahe des Randbereiches einer in der erwähnten Wandung vorhandenen Öffnung durch diese Wandung hindurchführen.

Zweckmäßigerweise sind in dem hinteren Fadenzuführungsteil und in dem vorderen Fadenaufnahmeteil m Gruppen mit jeweils m ≥ 2 von n nebeneinander liegenden Längs- und Aufnahmelöchern, mit n > 1 vorgesehen. Diese Maßnahme bringt den Vorteil mit sich, dass eine größere Anzahl n von Nähfäden in Gruppen m nahe des Randbereiches einer in der Wandung einer Hülle, eines Ballons oder einer Fläche, insbesondere einer Arterie eines Individuums vorhandenen Öffnung durch diese Wandung hindurchgeführt werden kann.

Bei einer besonders einfachen Vorrichtungskonstruktion sind die Führungsöffnungen des hinteren Fadenzuführungsteiles durch Längslöcher gebildet, in denen die an den Nadeln befestigten Nähfäden mittels gesonderter Schieber verschiebbar und über das mittlere Fadenfreigabe-/Fadenklemmteil in mit den Längslöchern fluchtende, die genannten Aufnahmeöffnungen bildende längliche Aufnahmelöcher des in seiner genannten einen Stellung befindlichen vorderen Aufnahmeteiles einführbar sind. Die betreffenden Schieber erlauben in vorteilhafter Weise ein sicheres und problemloses Hindurchführen der erwähnten Nadeln nahe des Randbereiches der in der Wandung einer Hülle, eines Ballons oder einer Fläche vorhandenen Öffnung durch die betreffende Wandung hindurch.

Dabei weisen die Aufnahmelöcher des vorderen Fadenaufnahmeteiles vorzugsweise eine solche Tiefe auf, dass zumindest die an den vorderen Enden der Nähfäden befindlichen Nadeln in diesen Aufnahmelöchern vollständig aufnehmbar sind. Die betreffenden Aufnahmelöcher können dabei vorzugsweise so ausgestaltet sein, dass sie die in sie einmal eingeführten Nadeln nur, wenn überhaupt, schwer herauszuziehen gestatten.

Um ein sicheres Festhalten der Nadeln in den erwähnten Aufnahmelöchern zu erreichen, können diese in dem vorderen Fadenaufnahmeteil zweckmäßigerweise mit ihrer jeweiligen Längsachse in bezug auf die Längsachse der Längslöcher des hinteren Fadenzuführungsteiles abgewinkelt verlaufen. Durch diese Maßnahme lassen sich die Nadeln in den betreffenden Aufnahmelöchern relativ einfach und sicher festklemmen.

Um die Nähfäden möglichst weit vom Randbereich der in der erwähnten Wandung vorhandenen Öffnung hindurchführen zu können, befinden sich die erwähnten Längslöcher vorzugsweise am Rand des Fadenzuführungsteiles und sind von einem an diesem Fadenzuführungsteil festgelegten Folienteil abgedeckt. Dies bringt den Vorteil eines besonders geringen konstruktiven Aufwands für die gewünschte Festlegung der Längslöcher mit sich.

Dabei sind die Längslöcher in dem hinteren Fadenzuführungsteil zweckmäßigerweise soweit zu dessen Außenumfang hin gelegt, dass ein Teil des Außenumfangs der Nadeln außerhalb des Außenumfangs des hinteren Fadenzuführungsteiles liegt. Diese Maßnahme unterstützt noch das Verlegen der Durchführungsöffnungen für die erwähnten Nähfäden möglichst weit vom Rand der erwähnten Öffnung weg.

Vorzugsweise befinden sich in dem Fadenzuführungsteil neben den Längslöchern Vorratskammern, in denen mit weiteren Nähfäden verbundene zusätzliche Nadeln untergebracht sind, die nach Einführen der in den genannten Längslöchern zunächst vorhandenen Nadeln in die in dem vorderen Fadenaufnahmeteil vorgesehenen Aufnahmelöcher und anschließendem Zurückziehen der für dieses Einführen vorgeschobenen Schieber in eine die betreffende Vorratskammern freigebende Rückzugsstellung in die genannten Längslöcher hinein gelangen, in welchen sie mittels der genannten Schieber in in dem vorderen Fadenaufnahmeteil vorhandene Aufnahmelöcher einführbar sind. Von dieser Maßnahme wird in vorteilhafter Weise dann Gebrauch gemacht, wenn mehr als zwei Nähfäden nahe des Randbereiches einer in der Wandung einer Hülle, eines Ballons oder einer Fläche, insbesondere einer Arterie eines Individuums vorhandenen Öffnung durch diese Wandung hindurchzuführen sind.

Für die Aufnahme der genannten weiteren Nadeln dienen zweckmäßigerweise dieselben Aufnahmelöcher in denen die Nadeln aufgenommen sind, welche sich zunächst in den Längslöchern befunden haben. Dazu können die betreffenden Aufnahmelöcher jeweils eine solche Länge aufweisen, dass sie zwei Nadeln hintereinander aufzunehmen gestatten. Es ist aber auch möglich, die betreffenden Aufnahmelöcher so auszugestalten, dass sie jeweils zwei Nadeln nebeneinander fest aufzunehmen vermögen.

Um die erwähnten weiteren Nadeln aus den genannten Vorratskammern auf einfache Weise herausführen zu können, wird eine Federkraft genutzt. Diese kann beispielsweise durch Druckfedern oder Spiralfedern erzeugt werden.

Vorzugsweise wird bei Vorhandensein von weiteren Aufnahmelöchern in dem vorderen Fadenaufnahmeteil vor der Einführung der in den zuvor erwähnten Vorratskammern untergebrachten weiteren Nadeln-in die genannten weiteren Aufnahmelöcher des vorderen Fadenaufnahmeteiles dieses eine solche relative Verdrehung zu dem hinteren Fadenzuführungsteil erfahren, dass die in dem vorderen Fadenaufnahmeteil vorhandenen weiteren Aufnahmelöcher zu den in dem hinteren Fadenzuführungsteil vorhandenen Längslöchern dann fluchten. Dadurch können beispielsweise vier Nähfäden in gleichen Abständen voneinander um den Randbereich der erwähnten Öffnung herum durch eine Wandung hindurchgeführt werden.

Zweckmäßigerweise erfahren das hintere Fadenzuführungsteil und das vordere Fadenaufnahmeteil vor der Einführung der genannten weiteren Nadeln in die Aufnahmelöcher des vorderen Fadenaufnahmeteiles eine relative Verdrehung gegenüber dem mittleren Fadenfreigabe-/Fadenklemmteil. Hierdurch ergibt sich der Vorteil, dass beispielsweise vier Nähfäden in gleichen Abständen voneinander um den Randbereich der erwähnten Öffnung herum durch eine Wandung hindurchgeführt werden.

Zweckmäßigerweise ist das mittlere Fadenfreigabe-/Fadenklemmteil mittels einer das hintere Fadenzuführungsteil drehbar durchziehenden Hülse mit einem Handgriff verbunden, und das hintere Fadenzuführungsteil und das vordere Fadenaufnahmeteil sind zweckmäßigerweise gegebenenfalls über eine koaxial zu der genannten Hülse angeordnete Hülsenanordnung mit Dreheinstellrädern verbunden. Dies bringt den Vorteil einer relativ einfachen Konstruktion für die Einstellung und Bewegung der einzelnen Vorrichtungsteile mit sich.

Die vorstehend erwähnten Dreheinstellräder sind zweckmäßigerweise mit Rastgesperren verbunden, die die betreffenden Dreheinstellräder und damit das mit diesen verbundene hintere Fadenzuführungsteil bzw. vordere Fadenaufnahmeteil in festgelegte Winkelpositionen relativ zu dem Handgriff und damit zu dem mittleren Fadenfreigabe-/Fadenklemmteil einzustellen gestatten. Hierdurch ergibt sich der Vorteil einer besonders einfach, aber dennoch wirksam einstellbaren Einstelleinrichtung für die einzelnen Vorrichtungsteile.

Zweckmäßigerweise sind die Dreheinstellräder für das hintere Fadenzuführungsteil und für das vordere Fadenaufnahmeteil mit einem Verriegelungs-/Freigabemechanismus derart gekoppelt, dass die Verschiebung der Nadeln durch den jeweiligen Schieber lediglich bei zueinander ausgerichteten Fadenzuführungsteil und Fadenaufnahmeteil freigegeben ist. Dies bringt den Vorteil mit sich, dass ein sicheres Verschieben der Nadeln durch den jeweiligen Schieber erst dann erfolgen kann, wenn das Fadenzuführungsteil und das Fadenaufnahmeteil zueinander ausgerichtet sind, was unter dem Gesichtspunkt einer Gefahren- bzw. Unfallverhütung von ganz besonderem Nutzen ist. Nur in dieser relativen Stellung des Fadenzuführungsteiles und des Fadenaufnahmeteiles ist sichergestellt, dass die Nadeln nicht in unbeabsichtigter oder fehlerhafter Weise aus der Vorrichtung herausgeführt werden können.

Von Vorteil dabei ist es, dass die Verschiebung der Nadeln in dem Fall freigegeben ist, dass sich das mittlere Fadenfreigabe-/Fadenklemmteil in seiner Fadenfreigabeposition befindet. Mit dieser Maßnahme ist gewährleistet, dass die genannten Nadeln bei zueinander ausgerichtetem Fadenzuführungsteil und Fadenaufnahmeteil erst dann verschoben werden können, wenn sich das dazwischen befindliche Fadenfreigabe-/Fadenklemmteil in seiner Fadenfreigabeposition befindet, also in der Position, die an sich zum Hindurchführen der Nadeln mit den damit verbundenen Nähfäden durch eine Wandung einer Hülle, eines Ballons oder einer Fläche, insbesondere einer Arterie eines Individuums vorgesehen ist.

Das hintere Fadenzuführungsteil und das vordere Fadenaufnahmeteil sind vorzugsweise von einem Führungselement durchzogen, welches der Einführung der gesamten Vorrichtung in die genannte Öffnung dient. Dabei ist zweckmäßigerweise mit Hilfe des betreffenden Führungselementes das vordere Fadenaufnahmeteil relativ zu dem hinteren Fadenzuführungsteil verdrehbar. Dies bringt den Vorteil mit sich, dass das betreffende Führungselement zusätzlich zu seiner Führungsaufgabe noch als Drehelement mit ausgenutzt werden kann. In diesem Fall kann das genannte Führungselement vorzugsweise durch einen Führungsdraht gebildet sein.

Bei dem gerade betrachteten Vorrichtungsaufbau ist das hintere Fadenzuführungsteil zweckmäßigerweise von einem das Führungselement umgebenden Hülsenteil durchzogen, durch welches das mittlere Fadenfreigabe-/Fadenklemmteil relativ zu dem hinteren Fadenzuführungsteil verdrehbar ist. Hierdurch ist bei der gerade betrachteten Vorrichtungskonstruktion eine relativ einfache Verdrehbarkeit der betrachteten Vorrichtungsteile ermöglicht.

Es ist schließlich auch noch möglich, dass das vordere Fadenaufnahmeteil auf seiner dem mittleren Fadenfreigabe-/Fadenklemmteil zugewandten Fläche auf- bzw. einklappbare Federelemente aufweist, die auf ein Verdrehen des vorderen Fadenaufnahmeteiles aus seiner Ausgangslage relativ zu dem mittleren Fadenfreigabe-/Fadenklemmteil in einer ersten Richtung derart aufklappen, dass die Oberfläche des betreffenden vorderen Fadenaufnahmeteiles in Richtung zu dem mittleren Fadenfreigabe-/Fadenklemmteil entsprechend vergrößert ist, und die auf ein Zurückführen des Fadenaufnahmeteiles in seine genannte Ausgangslage wieder in ihren Einklappzustand zurückbringbar sind. Hierdurch ergibt sich der Vorteil, dass auf relativ einfache Weise die Anlagefläche des vorderen Fadenaufnahmeteiles an der Innenseite der Wandung, durch deren Öffnung das vordere Fadenaufnahmeteil hindurchgeführt ist, vergrößert ist, was eine größere Sicherheit beim Gebrauch der Vorrichtung gemäß der Erfindung mit sich bringt.

Anhand von Zeichnungen wird die Erfindung nachstehend beispielsweise näher erläutert, in denen für gleiche oder einander entsprechende Teile bzw. Elemente jeweils gleiche Bezugszeichen verwendet sind.
- Fig. 1: zeigt in einer Schnittansicht eine die Erfindung verkörpernde Vorrichtung gemäß einer ersten Ausführungsform der Erfindung.
- Fig. 2: zeigt eine Draufsicht auf ein hinteres Fadenzuführungsteil der in Fig. 1 dargestellten Vorrichtung entsprechend der dort eingetragenen Schnittlinie A-A.
- Fig. 3: zeigt eine Draufsicht auf ein mittleres Fadenfreigabe-/Fadenklemmteil der in Fig. 1 dargestellten Vorrichtung entsprechend der dort eingetragenen Schnittlinie B-B.
- Fig. 4: zeigt eine Draufsicht auf ein vorderes Fadenaufnahmeteil der in Fig. 1 dargestellten Vorrichtung entsprechend der dort eingetragenen Schnittlinie C-C.
- Fig. 5: zeigt eine Draufsicht auf das mittlere Fadenfreigabe-/Fadenklemmteil und das vordere Fadenaufnahmeteil der Vorrichtung gemäß Fig. 1 in einer Ausgangsstellung.
- Fig. 6: zeigt die in Fig. 5 dargestellten Vorrichtungsteile in einer von der dortigen Drehstellung verschiedenen Drehposition.
- Fig. 7: zeigt die in Fig. 5 dargestellten Vorrichtungsteile in einer anderen Drehstellung.
- Fig. 8: zeigt die in Fig. 5 dargestellten Vorrichtungsteile in einer noch weiteren Drehposition.
- Fig. 9: zeigt die in Fig. 5 dargestellten Vorrichtungsteile wieder in ihrer Ausgangslage, nachdem zuvor Nadeln mit Nähfäden durch Löcher nahe des Randbereiches einer in einer angedeuteten Wandung vorhandenen Öffnung entsprechend den Drehstellungen der Vorrichtungsteile gemäß Fig. 7 und 8 hindurchgeführt worden sind.
- Fig. 10: zeigt eine Seitenansicht, in der veranschaulicht ist, dass das vordere Fadenaufnahmeteil mit aufund einklappbaren Federelementen versehen ist, von denen in Fig. 10 lediglich eines sichtbar ist.
- Fig. 11: zeigt eine Draufsicht auf die in Fig. 10 dargestellten Vorrichtungsteile mit den beiden aufgeklappten Federelementen.
- Fig. 12: zeigt eine Modifikation der in Fig. 1 dargestellten Vorrichtung.
- Fig. 13: zeigt eine Vorrichtung gemäß einer weiteren Ausführungsform der Erfindung.
- Fig. 14: zeigt eine Schnittansicht der in Fig. 13 dargestellten Vorrichtung längs der dort eingetragenen Schnittlinie D-D.
- Fig. 15: zeigt eine noch weitere Ausführungsform der Vorrichtung gemäß der Erfindung.
- Fig. 16: zeigt eine Schnittansicht der in Fig. 15 dargestellten Vorrichtung längs der dort eingetragenen Schnittlinie E-E.
- Fig. 17: zeigt eine noch weitere Ausführungsform der Vorrichtung gemäß der Erfindung in einer Teilschnittansicht.
- Fig. 18: zeigt eine Unteransicht einer zu einem Verriegelungs-/Freigabemechanismus gehörenden Drehplatte.

Bevor auf die in den Zeichnungen dargestellten Einzelheiten im einzelnen eingegangen wird, sei zunächst generell angemerkt, dass im Rahmen der vorliegenden Anmeldung üblicherweise im Zusammenhang mit der Zuführung und Aufnahme von Nähfäden bzw. Fäden gemeint ist, dass auch die mit den betreffenden Nähfäden bzw. Fäden verbundenen Nadeln in diese Vorgänge eingeschlossen sind.

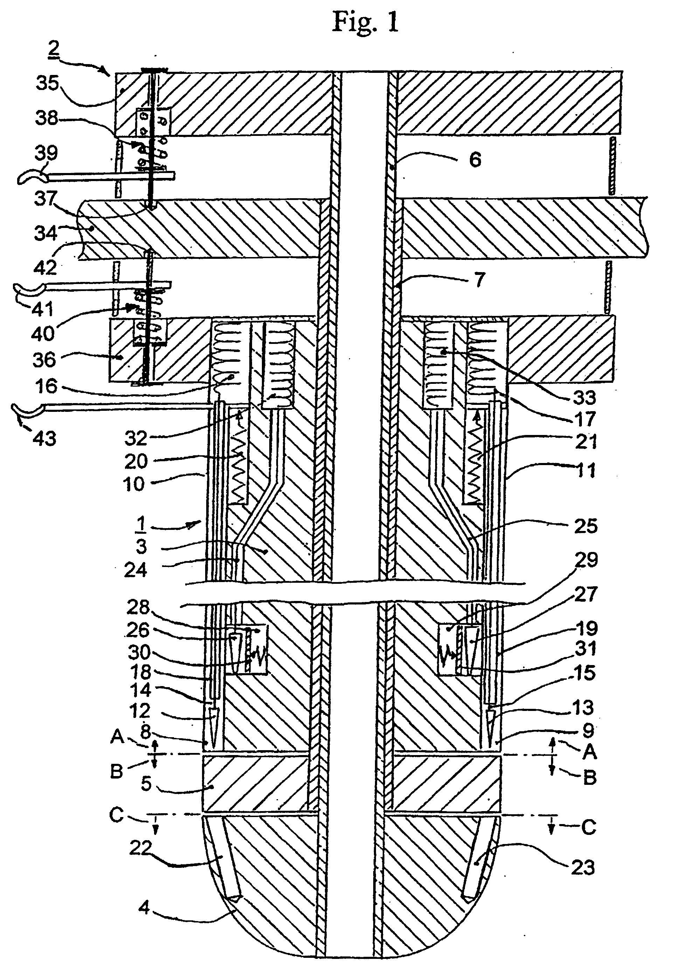

Fig. 1 zeigt in einer ausschnittweisen weitgehend rotationssymmetrischen Schnittansicht eine erste Ausführungsform einer Vorrichtung gemäß der Erfindung. Die in Fig. 1 dargestellte Vorrichtung besteht aus einer im unteren Teil der betreffenden Fig. 1 dargestellten Fadenführungseinrichtung 1 und aus einer im oberen Teil der Fig. 1 dargestellten Bedienungs- bzw. Betätigungseinrichtung 2, die eine Reihe von weiter unten noch näher erläuterten Bedienungs- bzw. Betätigungselementen aufweist. Diese Vorrichtung ist für den Fall, dass sie zum Verschließen einer in der Wand einer Arterie eines Individuums vorhandenen Öffnung dient, also als Arterien-Verschließvorrichtung eingesetzt wird, als stark vergrößert dargestellt zu betrachten. Im Falle einer solchen Arterien-Verschließvorrichtung beträgt die Abmessung der Fadenführungseinrichtung 1 in der Querschnittsrichtung lediglich einige wenige Millimeter; ein typischer Wert liegt beispielsweise bei 4 mm.

Zu der Fadenführungseinrichtung 1 gehören gemäß Fig. 1 in Längsrichtung der betreffenden Fadenführungseinrichtung betrachtet ein hinteres Fadenzuführungsteil 3, ein vorderes Fadenaufnahmeteil 4 und ein dazwischen liegendes mittleres Fadenfreigabe-/Fadenklemmteil 5. Diese drei Vorrichtungsteile 3, 4 und 5 sind im vorliegenden Fall über eine Doppelrohr- bzw. Doppelhülsenanordnung drehbar miteinander verbunden. Zu der betreffenden Hülsenanordnung gehören eine innere Hülse 6 und eine dazu koaxial angeordnete äußere Hülse 7. Die innere Hülse 6 ist gemäß Fig. 1 mit dem vorderen, das heißt am distalen Ende der drei Vorrichtungsteile 3, 4 und 5 vorgesehenen Fadenaufnahmeteil 4 fest verbunden. Die äußere Hülse 7 ist mit dem mittleren Fadenfreigabe-/Fadenklemmteil 5 fest verbunden und kann relativ zu der inneren Hülse 6 und auch relativ zu dem hinteren, das heißt am proximalen Ende der drei Vorrichtungsteile 4, 5 und 5 vorgesehenen Fadenzuführungsteil 3 verdreht werden. Dieses Verdrehen erfolgt durch verschiedene Elemente der oben erwähnten Betätigungseinrichtung 2, worauf weiter unten noch näher eingegangen wird.

Das mittlere Fadenfreigabe-/Fadenklemmteil 5 weist in dem Fall, dass die gesamte Vorrichtung eine Arterien-Verschließvorrichtung ist, eine Dicke auf, die einer Arterienwanddicke entspricht. Ein typischer Wert liegt bei ca. 2 mm. Die Länge des vorderen Nadelaufnahmeteiles 4 beträgt in diesem Fall typischerweise ca. 6 mm.

Das hintere Fadenzuführungsteil 3 weist zwei Längslöcher 8, 9 auf, die sich im vorliegenden Fall am Rand des Fadenzuführungsteiles 3 befinden und die dabei jeweils von einem Folienteil 10 bzw. 11 abgedeckt sind, das beispielsweise am Außenrand des Fadenzuführungsteiles 3 angeklebt sein kann. Dadurch gelingt es, die betreffenden Längslöcher 8, 9 möglichst weit nach außen zu verlegen, und zwar vorzugsweise soweit, dass ein Teil des Außenumfangs von in diesen Längslöchern 8, 9 aufgenommenen Nadeln 12, 13 außerhalb des Umfangs des hinteren Fadenzuführungsteiles 3 liegt. Dies ist von ganz besonderem Nutzen für den Fall, dass die erwähnten Nadeln möglichst weit von der Mitte der Fadenführungseinrichtung 1 zur Wirkung kommen sollen, was weiter unten noch näher ersichtlich werden wird.

Mit den zuvor erwähnten Nadeln 12, 13 sind Nähfäden 14, 15 verbunden, die jeweils von einem Nähfadenvorrat stammen, der in einem zugehörigen Nähfadenmagazin 16 bzw. 17 enthalten ist. Die erwähnten Nähfäden 14, 15 sind in den genannten Längslöchern 8, 9 durch Schieber 18, 19 hindurchgeführt, welche mittels einer Betätigungstaste 43 entgegen der Federkraft von Federn 20, 21 in Richtung zu dem vorderen Fadenaufnahmeteil 4 hin gedrückt werden können. Die betreffenden Schieber 18, 19 sind hier durch rohrförmige Schieber gebildet.

In dem vorderen Fadenaufnahmeteil 4 befinden sich Aufnahmelöcher 22, 23 für die Aufnahme der zuvor erwähnten Nadeln 12, 13. Die Aufnahmelöcher 22, 23 in dem vorderen Fadenaufnahmeteil 4 verlaufen dabei im vorliegenden Fall mit ihrer jeweiligen Eintrittsöffnung fluchtend zu den im hinteren Fadenzuführungsteil 3 vorhandenen Längslöchern 8 bzw. 9. Gemäß Fig. 1 sind die Aufnahmelöcher 22, 23 mit ihrer jeweiligen Längsachse in bezug auf die Längsachse der Längslöcher 8, 9 in Anpassung an die äußere Form des Fadenaufnahmeteiles 4 abgewinkelt. An dieser Stelle sei noch angemerkt, dass die Aufnahmeöffnungen 22, 23 jeweils eine solche Länge aufweisen, dass sie jeweils zwei Nadeln entsprechend den Nadeln 12 bzw. 13 hintereinander aufzunehmen vermögen. Dies bedeutet, dass die Aufnahmeöffnungen 22, 23 jeweils eine Länge aufweisen, die zumindest zweimal so groß ist wie die Länge jeder der Nadeln 12 bzw. 13. Im übrigen können die Aufnahmeöffnungen 22, 23 so ausgestaltet sein, dass die in sie jeweils eingeführte Nadel 12 bzw. 13 nicht ohne weiteres wieder herausgezogen werden kann. Dazu können an sich bekannte Verankerungsmechanismen in den Aufnahmeöffnungen 22, 23 vorgesehen sein.

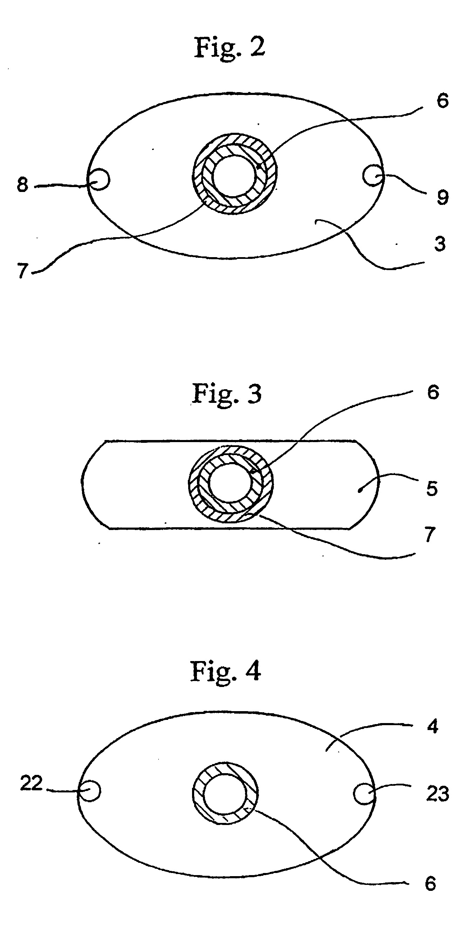

Bevor auf den Aufbau der in Fig. 1 dargestellten Vorrichtung weiter eingegangen wird, sollen zunächst die Querschnitte bzw. Formen des hinteren Fadenzuführungsteiles 3, des vorderen Fadenaufnahmeteiles 4 und des mittleren Fadenfreigabe-/Fadenklemmteiles 5 betrachtet werden. Die Querschnitte bzw. Formen dieser Vorrichtungsteile 3, 4 und 5 sind entsprechend den in Fig. 1 eingetragenen Schnittlinien A-A bzw. B-B bzw. C-C in Fig. 2, 3 und 4 veranschaulicht. Demgemäß weisen das hintere Fadenzuführungsteil 3, das vordere Fadenaufnahmeteil 4 und das mittlere Fadenfreigabe-/Fadenklemmteil 5 jeweils einen ovalförmigen Querschnitt bzw. eine ovale Form auf, wobei im vorliegenden Fall der Querschnitt bzw. die Form des mittleren Fadenfreigabe-/Fadenklemmteiles 5 gegenüber den Querschnitten bzw. Formen des hinteren Fadenzuführungsteiles 3 und des vorderen Fadenaufnahmeteiles 4 in der Breite vermindert ist, wie dies ein Vergleich der Fig. 3 mit den Fig. 2 und 4 klar erkennen läßt. Grundsätzlich weisen somit alle Vorrichtungsteile 3, 4 und 5 jeweils einen ovalförmigen bzw. elliptischen Querschnitt bzw. eine entsprechende Form auf, jedenfalls zumindest in den jeweils einander benachbarten Bereichen. Die Längslöcher 8, 9 und die Aufnahmelöcher 22, 23 befinden sich dabei jeweils an diametral gegenüberliegenden Stellen des Fadenzuführungsteiles 3 bzw. des Fadenaufnahmeteiles 4, und zwar auf der Längsachse des jeweiligen Formkörpers. Das Fadenfreigabe-/Fadenklemmteil 5 wirkt dabei in dem Fall, dass die in Fig. 1 dargestellte Vorrichtung zum Verschließen einer Arterienwandöffnung eingesetzt wird, als Wundrandspanner, der den Rand der eine Wunde darstellenden Arterienwandöffnung so spannt, dass in Querrichtung dieses Spannens die Nadeln 12, 13 bzw. die noch zu betrachtenden Nadeln 26, 27 mit ihren Fäden durch die Arterienwand hindurchgeführt werden können.

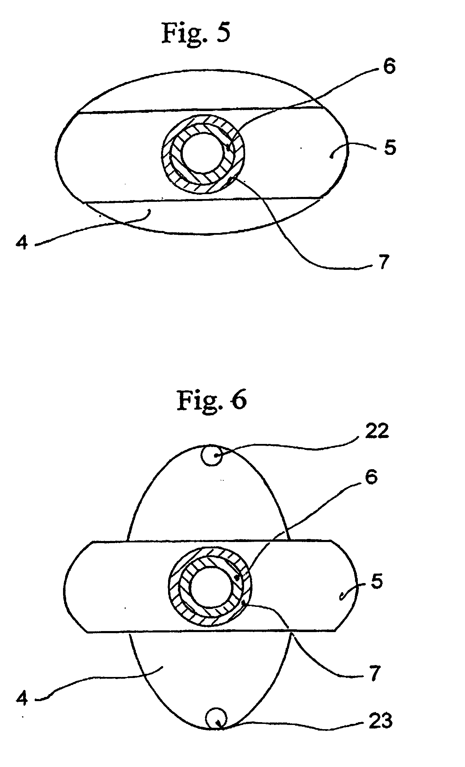

Die Fig. 5 und 6 zeigen in Draufsichten das mittlere Fadenfreigabe-/Fadenklemmteil 5 und das vordere Fadenaufnahmeteil 4 in zwei unterschiedlichen relativen Drehstellungen. In der Drehstellung gemäß Fig. 5, die als Ausgangsstellung der Vorrichtung betrachtet werden kann, sind die in dem vorderen Fadenaufnahmeteil 4 enthaltenen Aufnahmeöffnungen 22 und 23 durch das mittlere Fadenfreigabe-/Fadenklemmteil 5 abgedeckt. Das linke Fadenzuführungsteil 3 befindet sich dabei in derselben Drehstellung wie das vordere Fadenaufnahmeteil 4. In der in Fig. 6 dargestellten Drehposition sind die betreffenden Aufnahmeöffnungen 22, 23 frei zugänglich. Bezogen auf die in Fig. 1 dargestellte Vorrichtung heißt dies, dass im Falle einer Verdrehung des mittleren Fadenfreigabe-/Fadenklemmteiles 5 relativ zu dem vorderen Fadenaufnahmeteil 4 - diese relative Stellung ist in Fig. 6 veranschaulicht - die in den Längslöchern 8, 9 enthaltenen Nadeln 12 bzw. 13 zu den Aufnahmeöffnungen 22 bzw. 23 hin geführt werden können, da ihre Bewegung in dieser Position durch das mittlere Fadenfreigabe-/Fadenklemmteil 5 nicht behindert, sondern vielmehr freigegeben ist.

Zurückkommend auf die in Fig. 1 dargestellte Vorrichtung sei angemerkt, dass diese Vorrichtung im hinteren Fadenzuführungsteil 3 zusätzlich zu den bereits betrachteten Nadeln 12 und 13 mit zugehörigen Nähfäden noch mit weiteren Nähfäden 24 bzw. 25 verbundene zusätzliche Nadeln 26 bzw. 27 aufweist, die in Vorratskammern 28 bzw. 29 neben den erwähnten Längslöchern 8 bzw. 9 vorgesehen sind. In den betreffenden Vorratskammern 28, 29 sind durch Druckfedern belastete Druckplatten 30 bzw. 31 enthalten, durch die die zusätzlichen Nadeln 26 bzw. 27 in Richtung zu den erwähnten Längslöchern 8 bzw. 9 hin gedrückt werden. In der in Fig. 1 dargestellten Stellung der Vorrichtung hat diese Druckausübung noch keinen weiteren Effekt; die zusätzlichen Nadeln 26 bzw. 27 verbleiben in den Vorratskammern 28 bzw. 29. Erst wenn die Schieber 18 bzw. 19 nach erfolgter Einführung der in den Längslöchern 8 bzw. 9 zunächst vorhandenen Nadeln 12 bzw. 13 in die Aufnahmeöffnungen 22 bzw. 23 soweit zurückgezogen sind, dass die erwähnten Vorratskammern 28 bzw. 29 frei liegen, führt die erwähnte Druckausübung dazu, dass dann die zusätzlichen Nadeln 26 bzw. 27 in die erwähnten Längslöcher 8 bzw. 9 hinein gelangen, um anschließend durch die erwähnten Schieber 18 bzw. 19 in die Aufnahmeöffnungen 22 bzw. 23 hineingeschoben zu werden.

Die mit den zuvor erwähnten zusätzlichen Nadeln 26 bzw. 27 verbundenen weiteren Nähfäden 24 bzw. 25 gehören zu Nähfadenvorräten, die in gesonderten Nähfadenmagazinen 32 bzw. 33 in dem hinteren Fadenzuführungsteil 3 untergebracht sind.

Nunmehr sei die in Fig. 1 dargestellte Bedienungs- bzw. Betätigungseinrichtung 2 der Vorrichtung näher betrachtet. Zu dieser Betätigungseinrichtung 2 gehört zum einen ein Handgriff 34, der mit der oben erwähnten äußeren Hülse 7 der Vorrichtung fest verbunden ist. Mit diesem Handgriff kann die gesamte Vorrichtung gemäß Fig. 1 gehalten werden. Ferner gehört zu der Betätigungseinrichtung 2 ein Dreheinstellrad 35, welches mit der oben erwähnten inneren Hülse 6 fest verbunden ist, und außerdem gehört zu der betreffenden Betätigungseinrichtung 2 ein weiteres Dreheinstellrad 36, welches mit dem hinteren Fadenzuführungsteil 3 fest verbunden ist. Der Handgriff 34 weist auf seiner in Fig. 1 oben liegenden Seite an bestimmten Stellen Rastlöcher 37 auf, in die ein mit dem Dreheinstellrad 35 verbundenes Rastgesperre 38 einzugreifen vermag. Dieses lediglich schematisch durch eine federbelastete Stangenanordnung angedeutete Rastgesperre 38 ist mittels eines Betätigungshebels 39 aus seiner jeweiligen Rastposition heraushebbar, so dass nach diesem Herausheben eine Relativdrehung zwischen dem Dreheinstellrad 35 und dem Handgriff 34 möglich ist.

Auf seiner in Fig. 1 unten liegenden Seite weist der Handgriff 34 ebenfalls ein oder mehrere Rastlöcher 42 auf, in die ein ebenfalls nur schematisch als federbelastete Stangenanordnung angedeutetes Rastgesperre 40 einzugreifen vermag. Durch Betätigen eines Betätigungshebels 41 kann das Rastgesperre 40 aus der jeweiligen Rastöffnung 42 des Handgriffs herausgehoben werden, so dass danach eine Relativdrehung zwischen dem hinteren Fadenzuführungsteil 3 und dem Handgriff 34 ermöglicht ist.

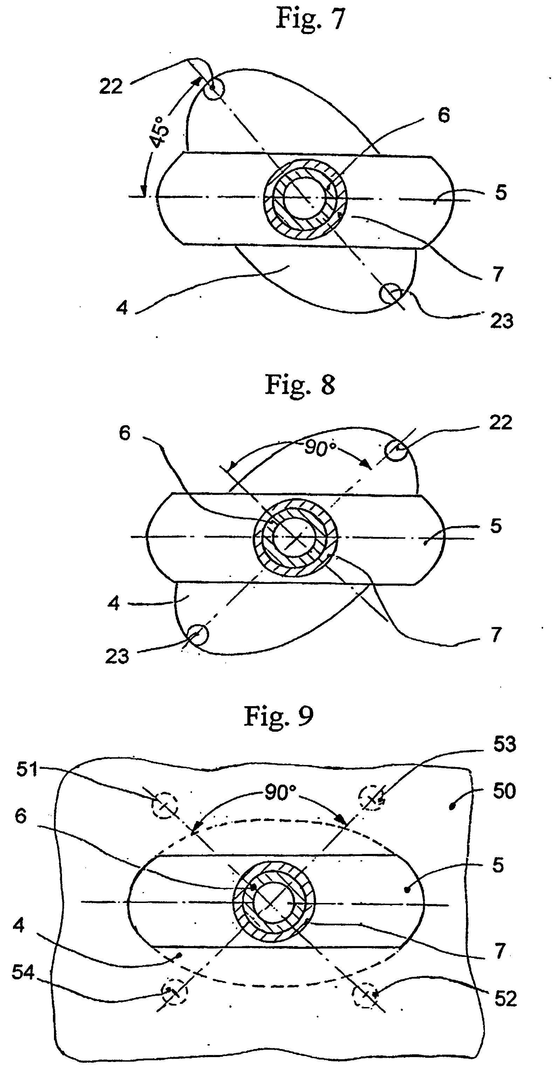

Wie im nachfolgenden noch ersichtlich werden wird, sind die Rastöffnungen 37 und 42 in dem Handgriff 34 jeweils an ganz bestimmten Stellen vorgesehen, die eine ganz gezielte Abgabe der in dem hinteren Fadenzuführungsteil 3 enthaltenen Nadeln 12, 13 bzw. 26, 27 ermöglichen, wie dies nachfolgend anhand der Fig. 7 bis 9 erläutert wird.

Fig. 7 zeigt die relative Verdrehung des vorderen Fadenaufnahmeteiles 4 in bezug auf das mittlere Fadenfreigabe-/Fadenklemmteil 5 in einer ersten Arbeitsstellung, in der die Aufnahmeöffnungen 22, 23 auf einer Geraden liegen, die gegenüber der in Längsrichtung des Fadenfreigabe-/Fadenklemmteiles 5 verlaufenden Längsachse einen Winkel von 45° einschließt. An dieser Stelle sei angemerkt, dass sich das hintere Fadenzuführungsteil 3 der Vorrichtung mit dem vorderen Fadenaufnahmeteil 4 in Deckung befindet. Dies bedeutet, dass die Längslöcher 8, 9 des hinteren Fadenzuführungsteiles in dieser Stellung bzw. Stichebene zu den Aufnahmeöffnungen 22, 23 des vorderen Fadenaufnahmeteiles 4 fluchten.

Fig. 8 zeigt die betreffende Vorrichtung in einer zweiten Arbeitsstellung bzw. Stichebene, in der das vordere Fadenaufnahmeteil 4 in bezug auf das mittlere Fadenfreigabe-/Fadenklemmteil 5 um 90° weiter gedreht ist. Es versteht sich, dass in dieser zweiten Arbeitsstellung auch das in Fig. 8 nicht dargestellte hintere Fadenzuführungsteil 3 sich in der gleichen Position bezogen auf das mittlere Fadenfreigabe-/Fadenklemmteil 5 befindet wie das vordere Fadenaufnahmeteil 4.

Während in der in Fig. 7 dargestellten ersten Arbeitsstellung der einzelnen Vorrichtungsteile die in Fig. 1 dargestellten Nadeln 12 und 13 mit ihren Fäden 14 und 15 von den AufnahmeÖffnungen 22 bzw. 23 aufgenommen werden, werden in der in Fig. 8 dargestellten zweiten Arbeitsstellung die zusätzlichen Nadeln 26 und 27 mit ihren Fäden 24 bzw. 25 in die Aufnahmeöffnungen 22 bzw. 23 eingeführt. Damit gelingt es, wie dies in Fig. 9 veranschaulicht ist, in einer Wandung 50 einer Hülle, eines Ballons oder einer Fläche, die insbesondere eine Arterie eines Individuums sein kann, in welchem bzw. in welcher sich eine Öffnung befindet, die sich mit ihrer Schnittfläche an den Außenumfang des mittleren Fadenfreigabe-/Fadenklemmteiles 5 anlegt, vier Nählöcher 51, 52, 53, 54 mit den betreffenden Nadeln zu durchstechen. Diese Nadeln sind dabei zusammen mit ihren Fäden in die Aufnahmeöffnungen 22 und 23 des vorderen Fadenaufnahmeteiles 4 eingeführt. Die Nählöcher 51, 52 sowie 53, 54 liegen jeweils auf einer Geraden, wobei die so gebildeten Geraden sich beispielsweise unter einem Winkel von 90° schneiden.

Den vorstehend anhand der Fig. 7 bis 9 erläuterten Drehpositionen sowie weiteren festgelegten Einstellpositionen (z.B. Ruhe- bzw. Ausgangsposition) des vorderen Fadenaufnahmeteiles 4 und des hinteren Fadenzuführungsteiles 3 relativ zu dem mittleren Fadenfreigabe-/Fadenklemmteil 4 entsprechende Rastlöcher sind in dem in Fig. 1 dargestellten Handgriff 34 vorgesehen. In Verbindung mit den oben erwähnten Rastgesperren 38 und 40 können damit die mit den Vorrichtungsteilen 3 und 4 verbundenen Einstellräder 35 bzw. 36 leicht in die jeweils gewünschten Drehpositionen gebracht werden.

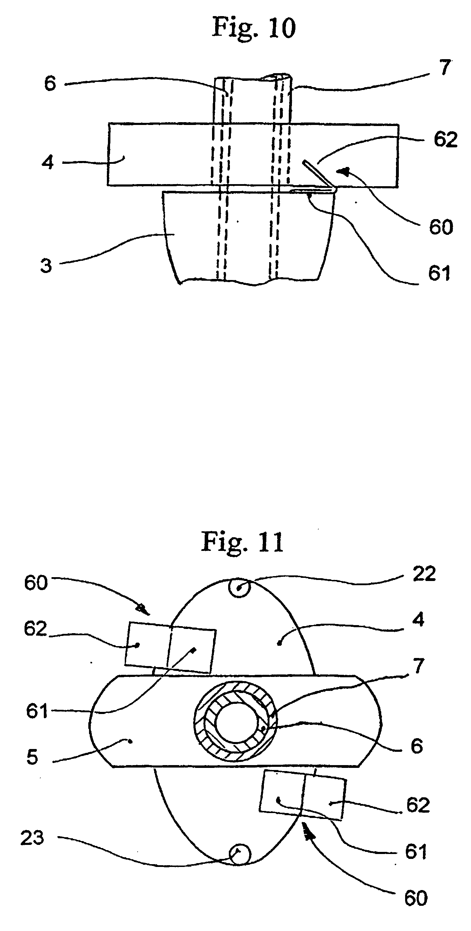

In Fig. 10 und 11 ist eine Ergänzung der vorstehend erläuterten Vorrichtung gemäß der Erfindung veranschaulicht. Gemäß dieser Ergänzung weist das vordere Fadenaufnahmeteil 3 auf seiner dem mittleren Fadenfreigabe-/Fadenklemmteil 4 zugewandten Fläche auf- und einklappbare Federelemente 60 auf, die im vorliegenden Fall - wie dies aus Fig. 11 deutlich hervorgeht - zweifach vorgesehen sind. Die beiden Federelemente 60 weisen jeweils eine an dem vorderen Fadenaufnahmeteil 4 angebrachte Befestigungsfläche 61 und aus- und einklappbare Flächen 62 auf. Die betreffenden Federelemente 60 sind dabei so gestaltet, dass auf ein Verdrehen des vorderen Fadenaufnahmeteiles 4 aus seiner Ausgangslage (wie sie beispielsweise in Fig. 5 veranschaulicht ist) relativ zu dem mittleren Fadenfreigabe-/Fadenklemmteil 5 in einer ersten Richtung aufklappen und damit die Oberfläche des vorderen Fadenaufnahmeteiles 4 in Richtung zu dem mittleren Fadenfreigabe-/Fadenklemmteil vergrößern. Auf ein Zurückdrehen des vorderen Fadenaufnahmeteiles 4 in seine genannte Ausgangslage hin sind die betreffenden Federelemente 60 mit ihren Flächen 62 wieder in den eingeklappten Zustand zurückzubringen. Durch diese Maßnahme der Bereitstellung von zusätzlichen Federelementen 60 kann somit die Anlagefläche des vorderen Fadenaufnahmeteiles 4 an einer Wandung, durch die das betreffende vordere Fadenaufnahmeteil 4 hindurchgesteckt ist, vergrößert werden. Dadurch ist gegebenenfalls eine sichere Arbeitsweise der betrachteten Vorrichtung gemäß der Erfindung ermöglicht.

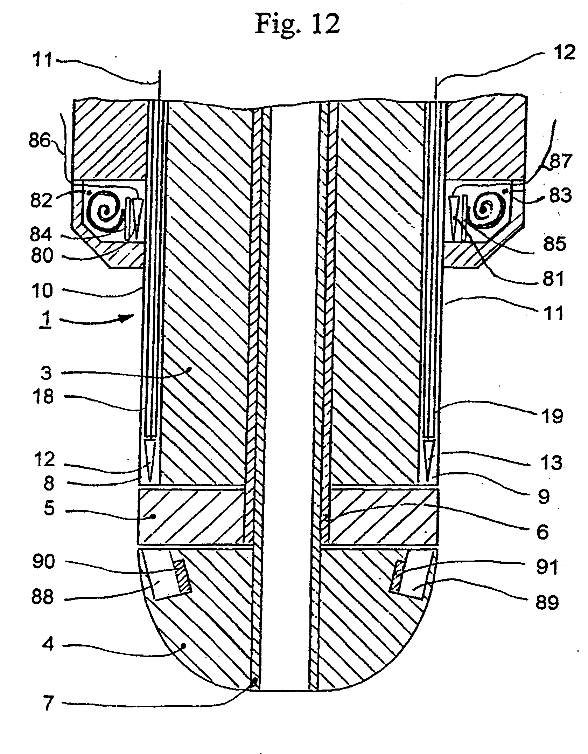

In Fig. 12 ist eine Modifikation der in Fig. 1 dargestellten Vorrichtung gemäß der Erfindung in einem Ausschnitt veranschaulicht. Gemäß Fig. 12 sind zusätzliche Nadeln 80, 81 in neben den Längslöchern 8 bzw. 9 auf der Außenseite der gesamten Vorrichtung gebildeten Vorratskammern 82 bzw. 83 untergebracht. Mit Hilfe von Druckplatten 84 bzw. 85, die mit Spiralfedern verbunden sind, wird ein Druck auf die betreffenden Nadeln 80 bzw. 81 in Richtung auf die genannten Längslöcher 8 bzw. 9 ausgeübt. Mit den erwähnten Nadeln 80 bzw. 81 sind hier auf der Außenseite der Vorrichtung verlaufende Fäden 86 bzw. 87 verbunden.

In dem vorderen Fadenaufnahmeteil 4 sind gemäß Fig. 12 im Unterschied zu den in Fig. 1 dargestellten Verhältnissen zwei Aufnahmeöffnungen 88, 89 vorgesehen, welche jeweils zwei Nadeln 12, 80 bzw. 13, 81 nebeneinander aufzunehmen vermögen. Um die zuerst in die Aufnahmeöffnungen 88, 89 eingeführten Nadeln 12 bzw. 13 aus der Einführungsbahn für die zusätzlichen zweiten Nadeln 80 bzw. 81 herauszubewegen, sind gemäß Fig. 12 für den Fall, dass zumindest die Nadeln 12, 13 magnetisch anziehbare Nadeln sind, in den Aufnahmeöffnungen 88 bzw. 89 Magnete 90 bzw. 91 vorgesehen. Diese Magnete ziehen die in die betreffenden Aufnahmeöffnungen eingeführten Nadeln 12 bzw. 13 an und halten sie fest.

Der übrige Aufbau und die sonstige Gestalt der in Fig. 12 dargestellten Vorrichtung stimmt mit der anhand von Fig. 1 erläuterten Vorrichtung überein.

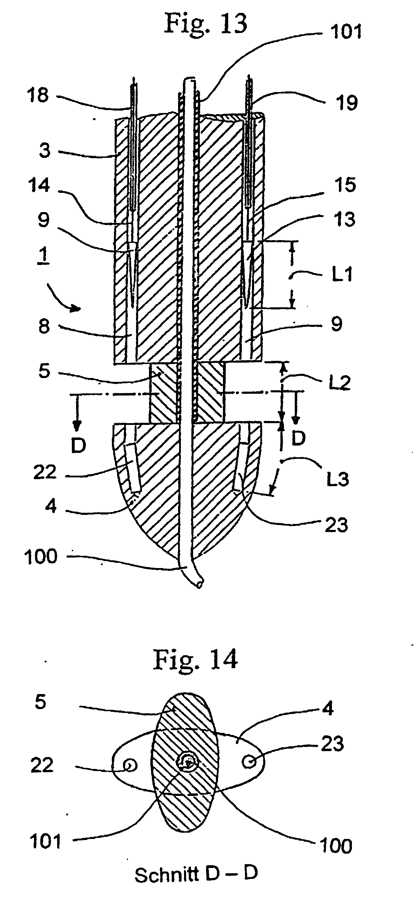

In Fig. 13 und 14 ist eine weitere Ausführungsform der Vorrichtung gemäß der vorliegenden Erfindung veranschaulicht. Diese Ausführungsform unterscheidet sich von der oben betrachteten Ausführungsform dadurch, dass die Vorrichtung lediglich zwei Nadeln 12, 13 enthält, mit denen Nähfäden 14 bzw. 15 verbunden sind. Im Unterschied zu der betrachteten Ausführungsform gemäß Fig. 1 oder 12 ist bei der Ausführungsform gemäß Fig. 13 und 14 jedoch der Querschnitt des hinteren Fadenzuführungsteiles 3, des vorderen Fadenaufnahmeteiles 4 und des mittleren Fadenfreigabe-/Fadenklemmteiles 5 jeweils ovalförmig ausgebildet, wobei genauer gesagt dieser ovalförmige Querschnitt zumindest in den angrenzenden Bereichen der jeweils benachbarten Teile vorgesehen ist.

Im Unterschied zu den in Fig. 1 und 12 näher dargestellten Verhältnissen sind bei der Ausführungsform gemäß Fig. 13 und 14 ein durch einen Führungsdraht 100 gebildetes Führungselement vorgesehen, welches mit dem vorderen Fadenaufnahmeteil 4 verbunden ist und dieses relativ zu dem hinteren Fadenzuführungsteil 3 und dem mittleren Fadenfreigabe-/Fadenklemmteil 5 zu verdrehen gestattet. Das erwähnte mittlere Fadenfreigabe-/Fadenklemmteil 5 ist mit einem Hülsenteil 101 fest verbunden, welches relativ zu dem Führungselement 100 und dem hinteren Fadenzuführungsteil 3 verdrehbar ist.

Im Zusammenhang mit der in Fig. 13 und 14 dargestellten Vorrichtung gemäß der Erfindung ist noch anzumerken, dass die in Fig. 13 eingetragene Länge L3 der Aufnahmeöffnungen 22 bzw. 23 zumindest so groß ist wie die Abmessung L1, das heißt die Länge einer der Nadeln 12 bzw. 13. Die Abmessung L2 entspricht zumindest der Dicke der Wandung, durch deren Öffnung die dargestellte Vorrichtung hindurchgeführt wird, um nahe deren Randbereich die Nadeln 12 bzw. 13 mit den damit verbundenen Fäden 14 bzw. 15 durch die betreffende Wandung hindurchführen zu können.

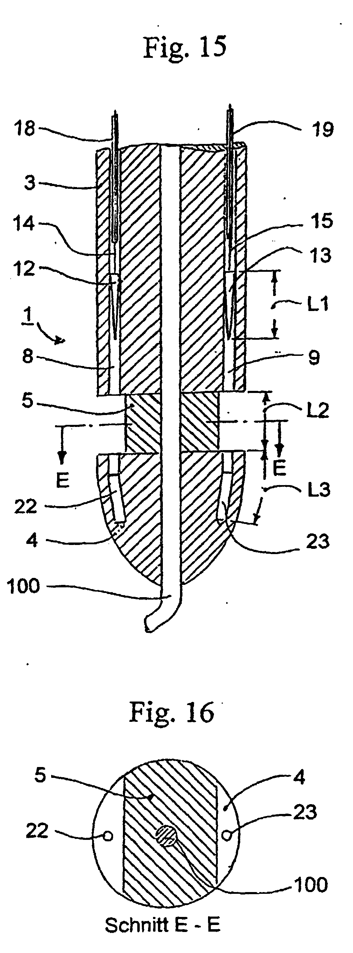

In Fig. 15 und 16 ist eine noch weitere Ausführungsform der Vorrichtung gemäß der vorliegenden Erfindung veranschaulicht. Diese einfachste Ausführungsform der hier erläuterten Ausführungsformen der Vorrichtung gemäß der Erfindung veranschaulicht, dass die vorliegende Erfindung prinzipiell auch dann funktioniert, wenn das hintere Fadenzuführungsteil 3 und das vordere Fadenaufnahmeteil 4 jeweils einen kreisförmigen Querschnitt aufweisen und wenn das mittlere Fadenfreigabe-/Fadenklemmteil 5 einen demgegenüber verminderten Querschnitt aufweist. Dabei kann es genügen, dass dieses mittlere Fadenfreigabe-/Fadenklemmteil 5 als Teil des hinteren Fadenzuführungsteiles 3 mit diesem fest verbunden oder sogar mit diesem zusammenhängend gebildet ist.

In Fig. 15 und 16 ist wie in den Fig. 13 und 14 ein sämtliche Vorrichtungsteile durchziehender Führungsdraht dargestellt, mit dessen Hilfe das vordere Fadenaufnahmeteil 4 relativ zu dem mittleren Fadenfreigabe-/Fadenklemmteil 5 und damit relativ zu dem hinteren Fadenzuführungsteil 3 verdrehbar ist. Durch diese Verdrehbarkeit werden auch im vorliegenden Fall die gleichen Funktionen realisiert, wie sie oben im Zusammenhang mit den Fig. 5 und 6 angesprochen worden sind.

Hinsichtlich der Längen L1, L2 und L3 gilt entsprechendes wie zu den Fig. 13 und 14 ausgeführt.

Abschließend sei noch angemerkt, dass die in Verbindung mit den Vorrichtungen gemäß Fig. 13 bis 16 erwähnten Führungselemente in Form von Führungsdrähten auch bei den in Fig. 1 bis 12 dargestellten Ausführungsformen vorgesehen sein können, allerdings dort lediglich als reine Führungselemente, mit denen keine Verdrehfunktion verbunden ist, wie dies in Verbindung mit Fig. 13 bis 16 erläutert worden ist.

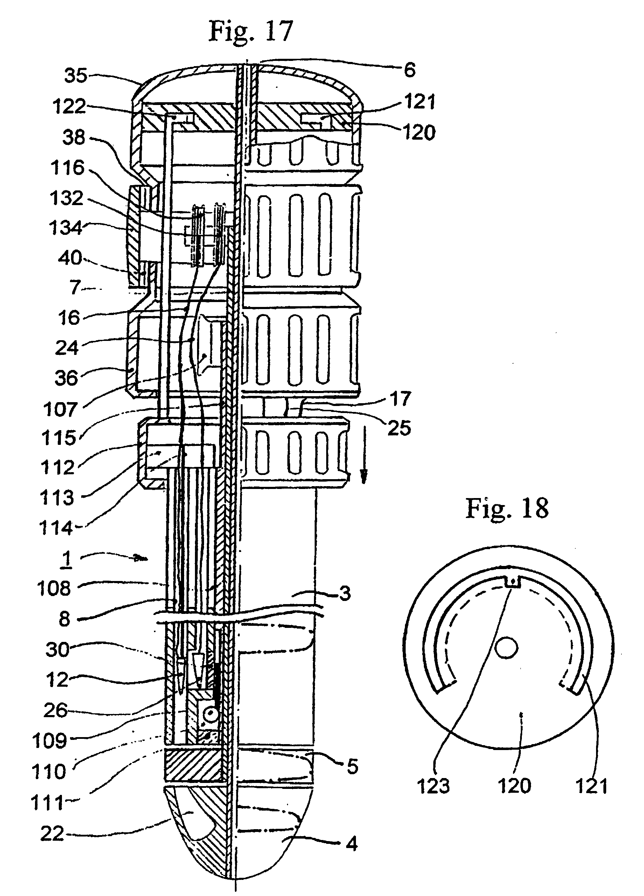

In Fig. 17 ist eine noch weitere Ausführungsform der Vorrichtung gemäß der Erfindung dargestellt. Für die betreffende Darstellung ist dabei weitgehend ein Halbschnitt gewählt, wobei allerdings im oberen Teil der Fig. 17 ein Vollschnitt vorgesehen ist. Die betreffende Darstellung ist dabei stark vergrößert, und zwar insbesondere im unteren Teil, um Einzelheiten zu verdeutlichen, auf die nachstehend näher eingegangen wird. Da durch die Halbschnittdarstellung nur der nähere Aufbau in der einen Hälfte der betreffenden Vorrichtung veranschaulicht ist, wird im folgenden auch nur dieser Aufbau näher beschrieben. Es versteht sich, dass die Vorrichtung in ihrer nicht im Schnitt dargestellten anderen Hälfte zumindest in weitgehend entsprechender Weise aufgebaut ist. Im übrigen sei noch angemerkt, dass grundsätzlich in Fig. 17 diejenigen Elemente, die den in Fig. 1 dargestellten Elementen mit vergleichbarer bzw. gleicher Funktion entsprechen, mit denselben Bezugszeichen bezeichnet sind, wie in Fig. 1.

Die in Fig. 17 dargestellte Vorrichtung 1 weist wie die in Fig. 1 dargestellte Vorrichtung in ihrer Fadenführungseinrichtung 1 ein Fadenzuführungsteil 3, ein Fadenfreigabe-/Fadenklemmteil 5 und ein Fadenaufnahmeteil 4 in der betreffenden Reihenfolge vom proximalen Ende zum distalen Ende der dargestellten Vorrichtung auf. Die betreffenden Teile 3, 4, 5 weisen dabei jeweils einen ovalförmigen bzw. elliptischen Querschnitt auf, wie dies in Fig. 17 durch Strichpunktlinien angedeutet ist. In dem Fadenzuführungsteil 3 befinden sich Längslöcher 8, 108 für die Aufnahme der mit den Nadeln 12 bzw. 26 verbundenen Nähfäden 16 bzw. 24 sowie entsprechende Längslöcher für die Aufnahme der mit nicht sichtbaren Nadeln verbundenen Nähfäden 17, 25. Die Nadel 26 ist dabei in einer Aufnahmekammer im unteren Bereich des Fadenzuführungsteiles 3 in entsprechender Weise aufgenommen, wie dies in Fig. 1 bereits gezeigt ist. Demgemäß wird mittels einer Druckplatte 30 ein in Fig. 17 nach links gerichteter Druck auf die Nadel 26 ausgeübt. Dieser Druck stammt von einer Feder 109, die in einer im unteren Teil des Fadenzuführungsteiles 3 gebildeten Aufnahmekammer 110 aufgenommen ist, welche gemäß Fig. 17 durch ein Verschlußteil 111 zum Fadenfreigabe-/Fadenklemmteil 5 hin verschlossen ist.

Das Fadenaufnahmeteil 4 weist gemäß Fig. 17 ebenfalls Aufnahmelöcher auf, von denen lediglich das Aufnahmeloch 22 näher veranschaulicht ist. Dieses Aufnahmeloch 22 und sein entsprechendes Aufnahmeloch im anderen Bereich der dargestellten Vorrichtung sind im vorliegenden Fall reusenförmig ausgebildet. Dies bringt den Vorteil mit sich, dass eine einmal in das betreffende Aufnahmeloch eingeführte Nadel nicht ohne weiteres aus dieser Aufnahmeöffnung wieder herausgezogen werden kann, nachdem sie sich - was üblicherweise der Fall ist - in diesem Aufnahmeloch nicht in der Lage weiterhin befinden wird, in der es eingeführt worden ist. Dies trifft insbesondere für den Fall zu, dass der mit der betreffenden Nadel verbundenen Nähfaden seitlich mit dieser Nadel in bezug auf deren Längsrichtung verbunden ist.

Am oberen, das heißt hier proximalen Ende des die Teile 3, 4, 5 umfassenden Vorrichtungsabschnitts ist ein Verschiebeteil 112 vorgesehen, das an seinem Innenumfang ein durch eine flache Platte gebildetes Schiebeelement 113 aufweist. Mit diesem Schiebeelement 113 ist ein insbesondere durch einen Federdraht 114 gebildeter Schieber fest verbunden, mit dessen Hilfe die in dem Längsloch 8 jeweils befindliche Nadel 12 bzw. 26 in Längsrichtung, das heißt in Fig. 17 nach unten, verschoben werden kann. Dazu ist das Verschiebeteil 112 in Längsrichtung des Fadenzuführungsteiles 3 verschiebbar, das heißt gemäß Fig. 17 nach unten. Zu diesem Zweck sind die die Nähfäden 16, 24 aufnehmenden Längslöcher 8 und 108 derart geschlitzt, dass sie das betreffende Schiebeelement 113 aufzunehmen vermögen. An dieser Stelle sei angemerkt, dass in der Praxis die Anordnung so ist, dass das betreffende Schiebeelement 113 rechtwinklig zu der in Fig. 17 dargestellten Position in Schlitze der die Längslöcher 8, 108 begrenzenden Materialteile eintritt. Die jeweilige Schlitztiefe ist dabei so gewählt, dass dadurch sichergestellt ist, dass die jeweilige Nadel 12, 26 in das Aufnahmeloch 22 hineingelangen kann.

Das vorstehend betrachtete Verschiebeteil 112 ist zwar, wie beschrieben, in Längsrichtung zum distalen Ende der dargestellten Vorrichtung hin verschiebbar; in Drehrichtung ist es indessen mit dem ihm unmittelbar benachbarten Dreheinstellrad 36 fest verbunden. Zu diesem Zweck ist gemäß Fig. 17 eine von einem Befestigungsteil 107 in dem Dreheinstellrad 36 aufgenommene äußere Hülse 115 gewissermaßen als Teil der Verlängerung des Fadenzuführungsteiles 3 vorgesehen.

Oberhalb des vorstehend betrachteten Einstellrades 36 ist gemäß Fig. 17 ein dem Handgriff 34 in Fig. 1 entsprechendes Einstellrad 134 vorgesehen, welches mit einer Hülse 7 fest verbunden ist, die im Unterschied zu den in Fig. 1 dargestellten Verhältnissen hier allerdings eine mittlere Hülse ist. In dem Dreheinstellrad 134 sind im vorliegenden Fall den in Fig. 1 dargestellten Nähfadenmagazinen 16, 32 entsprechende Nähfadenrollen 116, 132 drehbar angeordnet, von denen die erwähnten Nähfäden 16 bzw. 24 abgezogen werden können. Mit dem Dreheinstellrad 134 kann im vorliegenden Fall das Fadenfreigabe-/Fadenklemmteil 5 in bezug auf das Fadenzuführungsteil 3 und das Fadenaufnahmeteil 4 verdreht werden, was den Verhältnissen entspricht, die im Zusammenhang mit Fig. 1 bereits erläutert worden sind.

Im oberen Teil der Fig. 17 ist das auch in Fig. 1 vorgesehene Dreheinstellrad 35 gezeigt, welches mit der inneren Hülse 6 verbunden ist, über die das Fadenaufnahmeteil 4 relativ zu dem Fadenzuführungsteil 3 und dem Fadenfreigabe-/Fadenklemmteil 5 verdrehbar ist. Die innere Hülse 6 ist hier, wie auch in Fig. 1 dargestellt, eine Leerhülse, durch die ein Führungsdraht, wie der Führungsdraht 100 als Führungselement hindurchführbar ist.

Um die in Fig. 17 dargestellten Dreheinstellräder 35, 134, 36 relativ zueinander in jeweils gewünschte Stellungen rastbar einstellen zu können, sind wie bei der in Fig. 1 dargestellten Ausführungsform Rastgesperre 38 und 40 zwischen dem Dreheinstellrad 134 und den beiden benachbarten Dreheinstellräder 35 und 36 vorgesehen. Diese Rastgesperre sind gemäß Fig. 17 allerdings nicht in Längsrichtung der dargestellten Vorrichtung vorgesehen, sondern vielmehr in radialer Richtung zwischen den betreffenden Dreheinstellrädern.

Zusätzlich zu den vorstehend betrachteten Elementen, mit denen die in Fig. 17 dargestellte Vorrichtung im Prinzip der in Fig. 1 dargestellten Vorrichtung entspricht, ist gemäß Fig. 17 noch ein gesonderter Verriegelungs-/Freigabemechanismus vorgesehen, mit dessen Hilfe sichergestellt wird, dass die Dreheinstellräder 35 und 36 für das Fadenzuführungsteil 3 und für das Fadenaufnahmeteil 4 eine Verschiebung der Nadeln durch den jeweiligen Schieber lediglich dann freigeben, wenn das Fadenzuführungsteil 3 und das Fadenaufnahmeteil 4 zueinander ausgerichtet sind. Zu diesem Verriegelungs-/Freigabemechanismus gehören im vorliegenden Fall eine mit dem Dreheinstellrad 35 verbundene Drehplatte 120, die einen L-förmigen Ausschnitt 121 aufweist, in welchem eine Verriegelungsstange 122 aufgenommen ist, die mit dem Verschiebeteil 112 fest verbunden ist. In Fig. 18 ist die betreffende Drehplatte 120 von unten her betrachtet dargestellt. Dabei erkennt man, dass diese Drehplatte 120 an einer bestimmten Position, im vorliegenden Fall in ihrer mittleren Position, im Bereich des konzentrisch zur Außenseite der betreffenden Drehplatte verlaufenden L-förmigen Ausschnitts 121 eine Ausnehmung 123 aufweist, in deren Bereich die genannte Verriegelungsstange 122 aus ihrer Verriegelungsposition herausführbar, also freigebbar ist. Allein in dieser Position kann das Verschiebeteil 112 gemäß Fig. 17 nach unten verschoben werden. Diese Position ist, wie oben bereits erwähnt, jene Position, in der das Fadenzuführungsteil 3 und das Fadenaufnahmeteil 4 zueinander ausgerichtet sind, und in der überdies vorzugsweise auch das Fadenfreigabe-/Fadenklemmteil 5 in seiner Fadenfreigabeposition ist.

Der vorstehend betrachtete Verriegelungs-/Freigabemechanismus ist im Zusammenhang mit dem Einstechen der Nadeln in einer einzigen Stichebene erläutert worden. Diese Stichebene bzw. ihre Lage ist dabei durch die Festlegung der Ausnehmung 123 definiert. Es dürfte jedoch einzusehen sein, dass in dem Fall, dass mehrere Stichebenen vorgesehen sind, wie dies an Hand der Fig. 7 bis 9 beispielsweise erläutert worden ist, eine dieser Anzahl von Stichebenen entsprechende Anzahl von der Ausnehmung 123 entsprechenden Ausnehmungen an solchen Stellen des L-förmigen Ausschnitts 121 vorgesehen sein wird, die den erwähnten Stichebenen bzw. deren Lagen entsprechen. Dabei können die Winkel zwischen den einzelnen Stichebenen auch andere Werte aufweisen als dies im Zusammenhang mit den Fig. 7 bis 9 erläutert worden ist, beispielsweise jeweils einen Wert von 60°.

Ergänzend sei zu der in Fig. 17 dargestellten Vorrichtung 1 noch angemerkt, dass bei dieser Vorrichtung die in Längsrichtung verlaufenden Außenseiten der Dreheinstellräder 35, 36 und 134 praktisch eine gemeinsame Außenlinie aufweisen, während das Verschiebeteil 112 mit seiner Außenseite demgegenüber nach innen versetzt ist. In Abweichung davon können jedoch auch andere Konfigurationen angewandt werden.

Im Bereich zwischen dem Verschiebeteil 112 und dem diesem unmittelbar benachbarten Dreheinstellrad 36 sind in Fig. 17 zusätzlich zu den Nähfäden 16, 24 und der Verriegelungsstange 122 noch die Nähfäden 17, 25 angedeutet. Dabei ist der Bereich zwischen dem Verschiebeteil 112 und dem Dreheinstellrad 36 als offener Bereich dargestellt. In der Praxis kann dieser Bereich jedoch durch entsprechende Abschirmteile nach außen verschlossen sein, die beispielsweise teleskopartig mit dem Dreheinstellrad 36 und dem Verschiebeteil 112 verbunden sind.

Im übrigen ist noch darauf hinzuweisen, dass die Erfindung auf die beschriebenen Ausführungsformen nicht beschränkt ist, sondern dass in dem hinteren Fadenzuführungsteil 3 und in dem vorderen Fadenaufnahmeteil 4 jeweils m Gruppen mit m ≥ 2 von n nebeneinander liegenden Längs- und Aufnahmelöchern, mit n ≥ 1 vorgesehen sein können. Dadurch ergeben sich im Falle von m > 2 geometrische Anordnungen für die einzelnen Vorrichtungsteile, welche von den in den Zeichnungen dargestellten geometrischen Anordnungen abweichen. So ergibt sich beispielsweise im Falle von m = 3 eine dreieckige Querschnittskonfiguration für die Vorrichtung gemäß der Erfindung.

## Patentansprüche

1. Vorrichtung zum Hindurchführen wenigstens zweier Nähfäden (14, 15; 24, 25) durch eine Wandung einer Hülle, eines Ballons oder einer Fläche, insbesondere einer Arterie eines Individuums nahe des Randbereiches einer darin vorhandenen, gegebenenfalls durch Ein- und/oder Freischneiden gebildeten öffnung und zum Zurückziehen der durch die betreffende Wandung hindurchgeführten Nähfädenenden aus der genannten Öffnung, mit einer stabförmigen Fadenführungseinrichtung (1), in der die an Nadeln (12, 13; 26, 27) befestigten Nähfäden (14, 15; 24, 25) in Führungs- bzw. Aufnahmeöffnungen (8, 9; 22, 23) derart geführt sind, dass sie mit der betreffenden Fadenführungseinrichtung (1) durch die Wandung der Hülle, des Ballons oder der Fläche hindurchführbar und aus der genannten Öffnung wieder derart zurückziehbar sind , dass durch Zusammenziehen der Nähfädenenden außerhalb der betreffenden Öffnung diese verschließbar ist,
**dadurch gekennzeichnet,**
**dass** die Fadenführungseinrichtung (1) in ihrer Längsrichtung ein hinteres Fadenzuführungsteil (3), ein vorderes Fadenaufnahmeteil (4) und ein dazwischen liegendes mittleres Fadenfreigabe-/Fadenklemmteil (5) enthält
und **dass** das genannte mittlere Fadenfreigabe-/Fadenklemmteil (5) zumindest relativ zu dem vorderen Fadenaufnahmeteil (4) verdrehbar ist und einen solchen Querschnitt aufweist, dass es in zumindest einer Drehstellung die Einführung der von dem hinteren Fadenzuführungsteil (3) zugeführten Nähfäden (14, 15; 24, 25) in in dem vorderen Fadenaufnahmeteil (4) freiliegende Aufnahmeöffnungen (22, 23) ermöglicht und in einer von der genannten Drehstellung verschiedenen Drehposition die in der betreffenden Aufnahmeöffnungen (22, 23) zusammen mit den Nadeln (12, 13; 26, 27) aufgenommenen Nähfäden (14, 15; 24, 25) für ein Herausziehen der gesamten Fadenführungseinrichtung (1) aus der genannten Öffnung heraus festzuhalten gestattet.

2. Vorrichtung nach Anspruch 1, **dadurch gekennzeichnet, dass** das hintere Fadenzuführungsteil (3), das mittlere Fadenfreigabe-/Fadenklemmteil (5) und das vordere Fadenaufnahmeteil (4) jeweils einen ovalförmigen Querschnitt aufweisen.

3. Vorrichtung nach Anspruch 2, **dadurch gekennzeichnet, dass** das hintere Fadenzuführungsteil (3), das mittlere Fadenfreigabe-/Fadenklemmteil (5) und das vordere Fadenaufnahmeteil (4) der Fadenführungseinrichtung (1) zumindest in ihren angrenzenden Bereichen jeweils denselben ovalförmigen Querschnitt aufweisen.

4. Vorrichtung nach Anspruch 2, **dadurch gekennzeichnet, dass** der Querschnitt des mittleren Fadenfreigabe-/Fadenklemmteiles (5) gegenüber den Querschnitten des hinteren Fadenzuführungsteiles (3) und des vorderen Fadenaufnahmeteiles (4) in der Querschnittsbreite vermindert ist.

5. Vorrichtung nach einem der Ansprüche 1 bis 4, **dadurch gekennzeichnet, dass** das hintere Fadenzuführungsteil (3), das mittlere Fadenfreigabe-/Faden-klemmteil (5) und das vordere Fadenaufnahmeteil (4) der Fadenführungseinrichtung (1) alle relativ zueinander verdrehbar sind.

6. Vorrichtung nach Anspruch 1, **dadurch gekennzeichnet, dass** das mittlere Fadenfreigabe-/Fadenklemmteil (5) durch einen Bereich verminderten Querschnitts des hinteren Fadenzuführungsteils (3) gebildet ist.

7. Vorrichtung nach Anspruch 6, **dadurch gekennzeichnet, dass** der Bereich verminderten Querschnitts des hinteren Fadenzuführungsteiles (3) durch ein gesondertes Teil (5) gebildet ist, welches mit dem hinteren Fadenzuführungsteil (3) fest verbunden ist.

8. Vorrichtung nach einem der Ansprüche 1 bis 7, **dadurch gekennzeichnet, dass** das mittlere Fadenfreigabe-/ Fadenklemmteil (5) eine der Dicke der Wandung einer Hülle, eines Ballons oder einer Fläche, insbesondere einer Arterie eines Individuums entsprechende Dicke (L2) aufweist.

9. Vorrichtung nach einem der Ansprüche 1 bis 8, **dadurch gekennzeichnet, dass** in dem hinteren Fadenzuführungsteil (3) und in dem vorderen Fadenaufnahmeteil (4) m Gruppen, mit m ≥ 2 von n nebeneinander liegenden Längs- und Aufnahmelöchern (8, 9; 22, 23), mit n ≥ 1 vorgesehen sind.

10. Vorrichtung nach einem der Ansprüche 1 bis 9, **dadurch gekennzeichnet, dass** das hintere Fadenzuführungsteil (3) Längslöcher (8, 9) aufweist, in denen die an den Nadeln (12, 13; 26, 27) befestigten Nähfäden (14, 15; 24, 25) mittels gesonderter Schieber (18, 19) verschiebbar und über das mittlere Fadenfreigabe-/Fadenklemmteil (5) in mit den Längslöchern (8, 9) fluchtende Aufnahmelöcher (22, 23) des in seiner genannten einen Stellung befindlichen vorderen Fadenaufnahmeteiles (4) einführbar sind.

11. Vorrichtung nach Anspruch 10, **dadurch gekennzeichnet, dass** die Aufnahmelöcher (22, 23) des vorderen Fadenaufnahmeteiles (4) eine solche Tiefe (L3) aufweisen, dass zumindest die an den den vorderen Enden der Nähfäden (14, 15; 24, 25) befindlichen Nadeln (12, 13; 26, 27) in diesen Aufnahmelöchern (22, 23) vollständig aufnehmbar sind.

12. Vorrichtung nach Anspruch 10 oder 11, **dadurch gekennzeichnet, dass** die Aufnahmelöcher (22, 23) im vorderen Bereich des vorderen Fadenaufnahmeteiles (4) in Bezug auf ihre mit der Längsachse der Längslöcher (8, 9) des hinteren Fadenzuführungsteiles (3) fluchtende Längsachse abgewinkelt verlaufen.

13. Vorrichtung nach einem der Ansprüche 10 bis 12, **dadurch gekennzeichnet, dass** die Führungsöffnungen (8, 9) sich am Rand des Fadenzuführungsteiles (3) befinden und von einem an diesem Fadenzuführungsteil (3) festgelegten Folienteil (10, 11) abgedeckt sind.

14. Vorrichtung nach Anspruch 13, **dadurch gekennzeichnet, dass** die Führungsöffnungen (8, 9) in dem hinteren Fadenzuführungsteil (3) soweit zu dessen Außenumfang hin gelegt sind, dass ein Teil des Außenumfangs der Nadeln (12, 13; 26, 27) außerhalb des Außenumfangs des hinteren Fadenzuführungsteiles (3) liegt.

15. Vorrichtung nach einem der Ansprüche 10 bis 14, **dadurch gekennzeichnet, dass** sich in dem Fadenzuführungsteil (3) neben den Führungsöffnungen (8, 9) Vorratskammern (28, 29) befinden, in denen mit weiteren Nähfäden (24, 25) verbundene zusätzliche Nadeln (26, 27) untergebracht sind, die nach Einführen der in den genannten Führungsöffnungen (8, 9) zunächst vorhandenen Nadeln (12, 13) in die in dem vorderen Fadenaufnahmeteil (4) vorgesehenen Aufnahmeöffnungen (22, 23) und anschließendem Zurückziehen der für dieses Einführen vorgeschobenen Schieber (18, 19) in eine die betreffenden Vorratskammern (28, 29) freigebende Rückzugsstellung in die genannten Führungsöffnungen (8, 9) hinein gelangen, in welchen sie mittels der genannten Schieber (18, 19) in in dem vorderen Fadenaufnahmeteil (4) vorhandene Aufnahmeöffnungen (22, 23) einführbar sind.

16. Vorrichtung nach Anspruch 13, **dadurch gekennzeichnet, dass** für die Aufnahme der genannten weiteren Nadeln (26, 27) dieselben Aufnahmeöffnungen (22, 23) dienen, in denen die Nadeln (12, 13) aufgenommen sind, welche sich zunächst in den Führungsöffnungen (8, 9) befunden haben.

17. Vorrichtung nach Anspruch 14 oder 15, **dadurch gekennzeichnet, dass** die in den Vorratskammern (28, 29) untergebrachten zusätzlichen Nadeln (26, 27) durch Federkraft in die genannten Führungsöffnungen (8, 9) einführbar sind.

18. Vorrichtung nach einem der Ansprüche 14 bis 17, **dadurch gekennzeichnet, dass** bei Vorhandensein von weiteren Aufnahmelöchern in dem vorderen Fadenaufnahmeteil (4) vor der Einführung der in den Vorratskammern (28, 29) untergebrachten weiteren Nadeln (26, 27) in die genannten weiteren Aufnahmelöcher des vorderen Fadenaufnahmeteiles (4) dieses eine solche relative Verdrehung zu dem hinteren Fadenzuführungsteil (3) erfährt, dass die in dem vorderen Fadenaufnahmeteil (4) vorhandenen weiteren Aufnahmelöcher zu den in dem hinteren Fadenzuführungsteil (3) vorhandenen Führungsöffnungen (8, 9) fluchten.

19. Vorrichtung nach einem der Ansprüche 15 bis 18, **dadurch gekennzeichnet, dass** das hintere Fadenzuführungsteil (3) und das vordere Fadenaufnahmeteil (4) vor der Einführung der genannten weiteren Nadeln (26, 27) in die Aufnahmeöffnungen (22, 23) des vorderen Fadenaufnahmeteiles (4) eine relative Verdrehung gegenüber dem mittleren Fadenfreigabe-/Fadenklemmteil (5) erfahren.

20. Vorrichtung nach einem der Ansprüche 1 bis 19, **dadurch gekennzeichnet, dass** das mittlere Fadenfreigabe-/Fadenklemmteil (5) mittels eines das hintere Fadenzuführungsteil (3) drehbar durchziehenden Hülsenteiles (7) mit einem Handgriff (34) verbunden ist und dass das hintere Fadenzuführungsteil (3) und das vordere Fadenaufnahmeteil (4) gegebenenfalls über eine koaxial zu dem genannten Hülsenteil (7) angeordnete Hülsenanordnung (6) mit Dreheinstellrädern (35, 36) verbunden sind.

21. Vorrichtung nach Anspruch 20, **dadurch gekennzeichnet, dass** die Dreheinstellräder (35, 36) mit Rastgesperren (38, 40) verbunden sind, die die betreffenden Dreheinstellräder (35, 36) und damit das mit diesen verbundene hintere Fadenzuführungsteil (3) bzw. vordere Fadenaufnahmeteil (4) in festgelegte Winkelpositionen relativ zu dem Handgriff (34) und damit zu dem mittleren Fadenfreigabe-/Fadenklemmteil (5) einzustellen gestatten.

22. Vorrichtung nach Anspruch 20 oder 21, **dadurch gekennzeichnet, dass** die Dreheinstellräder (35, 36) für das hintere Fadenzuführungsteil (3) und für das vordere Fadenaufnahmeteil (4) mit einem Verriegelungs-/Freigabemechanismus (120 bis 123) derart gekoppelt sind, dass die Verschiebung der Nadeln (12, 13; 26, 27) durch den jeweiligen Schieber (18, 19; 114) lediglich bei zueinander ausgerichteten Fadenzuführungsteil (3) und Fadenaufnahmeteil (4) freigegeben ist.

23. Vorrichtung nach Anspruch 22, **dadurch gekennzeichnet, dass** die Verschiebung der Nadeln (12, 13; 26, 27) in dem Fall freigegeben ist, dass sich das mittlere Fadenfreigabe-/Fadenklemmteil (5) in seiner Fadenfreigabeposition befindet.

24. Vorrichtung nach einem der Ansprüche 1 bis 23, **dadurch gekennzeichnet, dass** das hintere Fadenzuführungsteil (3) und das vordere Fadenaufnahmeteil (4) von einem Führungselement (100) durchzogen sind, mit dessen Hilfe das vordere Fadenaufnahmeteil (4) relativ zu dem hinteren Fadenzuführungsteil (3) verdrehbar ist.

25. Vorrichtung nach Anspruch 24, **dadurch gekennzeichnet, dass** das Führungselement (100) durch einen Führungsdraht (100) gebildet ist.

26. Vorrichtung nach Anspruch 24 oder 25, **dadurch gekennzeichnet, dass** das hintere Fadenzuführungsteil (3) von einem das Führungselement (100) umgebenden Hülsenteil (101) durchzogen ist, durch das das mittlere Fadenfreigabe-/Fadenklemmteil (5) relativ zu dem hinteren Fadenzuführungsteil (3) verdrehbar ist.

27. Vorrichtung nach einem der Ansprüche 1 bis 26, **dadurch gekennzeichnet, dass** das vordere Fadenaufnahmeteil (4) auf seiner dem mittleren Fadenfreigabe-/ Fadenklemmteil (5) zugewandten Fläche auf- und einklappbare Federelemente (60) aufweist, die auf ein Verdrehen des vorderen Fadenaufnahmeteiles (4) aus seiner Ausgangslage relativ zu dem mittleren Fadenfreigabe-/Fadenklemmteil (5) in einer ersten Richtung derart aufklappen, dass die Oberfläche des betreffenden vorderen Fadenaufnahmeteiles (4) in Richtung zu dem mittleren Fadenfreigabe-/Fadenklemmteil (5) entsprechend vergrößert ist, und die auf ein Zurückführen Fadenaufnahmeteiles (4) in seine genannte Ausgangslage wieder in ihren eingeklappten Zustand zurückbringbar sind.

## Claims

1. Arrangement for guiding at least two sutures (14, 15; 24, 25) through a wall of a membrane, of a balloon or of a surface, in particular of an artery of an individual, in the vicinity of the edge region of an opening provided therein, and formed if appropriate by cutting in and/or cutting out, and for drawing back out of the abovementioned opening the suture ends guided through the relevant wall, having a shaft-like suture-guide device (1) in which the sutures (14, 15; 24, 25) fastened on needles (12, 13; 26, 27) are guided in guide and/or accommodating openings (8, 9; 22, 23) such that, by way of the relevant suture-guide device (1), they can be guided through the wall of the membrane, of the balloon or of the surface and drawn back out of the abovementioned opening again such that, by virtue of the suture ends being drawn together outside the relevant opening, the latter can be closed,
**characterized in that** the suture-guide device (1), in its longitudinal direction, contains a rear suture-feed part (3), a front suture-accommodating part (4) and a central suture-release/suture-clamping part (5) located there between, and **in that** the abovementioned central suture-release/suture-clamping part (5) can be rotated at least relative to the front suture-accommodating part (4) and has such a cross section that, in at least one rotary position, it allows the sutures (14, 15; 24, 25) fed from the rear suture-feed part (3) to be introduced into accommodating openings (22, 23) exposed in the front suture-accommodating part (4) and, in a rotary position differing from the abovementioned rotary position, it allows the sutures (14, 15; 24, 25) accommodated in the relevant accommodating openings (22, 23) together with the needles (12, 13; 26, 27) to be secured for drawing the entire suture-guide device (1) out of the abovementioned opening.

2. Arrangement according to Claim 1, **characterized in that** the rear suture-feed part (3), the central suture-release/suture-clamping part (5) and the front suture-accommodating part (4) each have an oval-shaped cross section.

3. Arrangement according to Claim 2, **characterized in that** the rear suture-feed part (3), the central suture-release/suture-clamping part (5) and the front suture-accommodating part (4) of the suture-guide device (1) each have the same oval-shaped cross section at least in their adjacent regions.

4. Arrangement according to Claim 2, **characterized in that** the cross section of the central suture-release/suture-clamping part (5) has a smaller width than the cross sections of the rear suture-feed part (3) and of the front suture-accommodating part (4).

5. Arrangement according to one of Claims 1 to 4, **characterized in that** the rear suture-feed part (3), the central suture-release/suture-clamping part (5) and the front suture-accommodating part (4) of the suture-guide device (1) can all be rotated relative to one another.

6. Arrangement according to Claim 1, **characterized in that** the central suture-release/suture-clamping part (5) is formed by a region of reduced cross section of the rear suture-feed part (3).

7. Arrangement according to Claim 6, **characterized in that** the region of reduced cross section of the rear suture-feed part (3) is formed by a separate part (5), which is fixed to the rear suture-feed part (3).

8. Arrangement according to one of Claims 1 to 7, **characterized in that** the central suture-release/suture-clamping part (5) has a thickness (L2) which corresponds to the thickness of the wall of a membrane, of a balloon or of a surface, in particular of an artery of an individual.

9. Arrangement according to one of Claims 1 to 8, **characterized in that** provided in the rear suture-feed part (3) and in the front suture-accommodating part (4) are m groups, where m ≥ 2, of n longitudinal and accommodating holes (8, 9; 22, 23) located one beside the other, where n ≥ 1.

10. Arrangement according to one of Claims 1 to 9, **characterized in that** the rear suture-feed part (3) has longitudinal holes (8, 9) in which the sutures (14, 15; 24, 25) fastened on the needles (12, 13; 26, 27) can be displaced by means of separate pushers (18, 19) and can be introduced, via the central suture-release/suture-clamping part (5), into accommodating holes (22, 23) aligned with the longitudinal holes (8, 9), said accommodating holes belonging to the front suture-accommodating part (4) located in its one position mentioned above.

11. Arrangement according to Claim 10, **characterized in that** the accommodating holes (22, 23) of the front suture-accommodating part (4) have such a depth (L3) that at least the needles (12, 13; 26, 27) located at the front end of the sutures (14, 15; 24, 25) can be accommodated in their entirety in said accommodating holes (22, 23).

12. Arrangement according to Claim 10 or 11, **characterized in that** the accommodating holes (22, 23) in the front region of the front suture-accommodating part (4) have their longitudinal axis, aligned with the longitudinal axis of the longitudinal slots (8, 9) of the rear suture-feed part (3), running at an angle.

13. Arrangement according to one of Claims 10 to 12, **characterized in that** the guide openings (8, 9) are located at the edge of the suture-feed part (3) and are covered by a sheeting part (10, 11) secured on said suture-feed part (3).

14. Arrangement according to Claim 13, **characterized in that** the guide openings (8, 9) in the rear suture-feed part (3) are positioned in the direction of the outer circumference thereof to such an extent that part of the outer circumference of the needles (12, 13; 26, 27) is located outside the outer circumference of the rear suture-feed part (3).

15. Arrangement according to one of Claims 10 to 14, **characterized in that** located in the suture-feed part (3), alongside the guide openings (8, 9), are supply chambers (28, 29) in which there are accommodated additional needles (26, 27) which are connected to further sutures (24, 25) and, once the needles (12, 13) initially provided in the abovementioned guide openings (8, 9) have been introduced into the accommodating openings (22, 23) provided in the front suture-accommodating part (4) and the pushers (18, 19) advanced for this introduction operation have subsequently been drawn back into a withdrawal position, in which the relevant supply chambers (28, 29) are released, pass into the abovementioned guide openings (8, 9), in which they can be introduced, by means of the abovementioned pushers (18, 19), into accommodating openings (22, 23) provided in the front suture-accommodating part (4).

16. Arrangement according to Claim 13, **characterized in that**, for accommodating the abovementioned further needles (26, 27), use is made of the same accommodating openings (22, 23) in which the needles (12, 13) which were initially located in the guide openings (8, 9) are accommodated.

17. Arrangement according to Claim 14 or 15, **characterized in that** the additional needles (26, 27) accommodated in the supply chambers (28, 29) can be introduced by spring force into the abovementioned guide openings (8, 9).

18. Arrangement according to one of Claims 14 to 17, **characterized in that** if further accommodating holes are provided in the front suture-accommodating part (4), before the further needles (26, 27) accommodated in the supply chambers (28, 29) are introduced into the abovementioned further accommodating holes of the front suture-accommodating part (4), the latter is rotated relative to the rear suture-feed part (3) such that the further accommodating holes provided in the front suture-accommodating part (4) are aligned in relation to the guide openings (8, 9) provided in the rear suture-feed part (3).

19. Arrangement according to one of Claims 15 to 18, **characterized in that**, before the abovementioned further needles (26, 27) are introduced into the accommodating openings (22, 23) of the front suture-accommodating part (4), the rear suture-feed part (3) and the front suture-accommodating part (4) are rotated relative to the central suture-release/suture-clamping part (5).

20. Arrangement according to one of Claims 1 to 19, **characterized in that** the central suture-release/suture-clamping part (5) is connected to a hand grip (34) by means of a sleeve part (7) which passes through the rear suture-feed part (3) in a rotatable manner, and **in that** the rear suture-feed part (3) and the front suture-accommodating part (4) are connected to rotary adjustment wheels (35, 36), if appropriate, via a sleeve arrangement (6) arranged coaxially with the abovementioned sleeve part (7).

21. Arrangement according to Claim 20, **characterized in that** the rotary adjustment wheels (35, 36) are connected to latching catches (38, 40) which allow the relevant rotary adjustment wheels (35, 36), and thus the rear suture-feed part (3) and front suture-accommodating part (4) connected thereto, to be adjusted into determined angle positions relative to the hand grip (34), and thus to the central suture-release/suture-clamping part (5).

22. Arrangement according to Claim 20 or 21, **characterized in that** the rotary adjustment wheels (35, 36) for the rear suture-feed part (3) and for the front suture-accommodating part (4) are coupled to a locking/release mechanism (120 to 123) such that the displacement of the needles (12, 13; 26, 27) by the respective pusher (18, 19; 114) is released only with the suture-feed part (3) and suture-accommodating part (4) aligned in relation to one another.

23. Arrangement according to Claim 22, **characterized in that** the displacement of the needles (12, 13; 26, 27) is released in the case where the central suture-release/suture-clamping part (5) is located in its suture-release position.

24. Arrangement according to one of Claims 1 to 23, **characterized in that** the rear suture-feed part (3) and the front suture-accommodating part (4) have a guide element (100) passing through them, with the aid of which the front suture-accommodating part (4) can be rotated relative to the rear suture-feed part (3).

25. Arrangement according to Claim 24, **characterized in that** the guide element (100) is formed by a guide wire (100).

26. Arrangement according to Claim 24 or 25, **characterized in that** the rear suture-feed part (3) has a sleeve part (101), which encloses the guide element (100), passing through it, it being possible for the central suture-release/suture-clamping part (5) to be rotated relative to the rear suture-feed part (3) by means of said sleeve part.

27. Arrangement according to one of Claims 1 to 26, **characterized in that**, on its surface directed towards the central suture-release/suture-clamping part (5), the front suture-accommodating part (4) has swing-open and swing-in spring elements (60) which, with rotation of the front suture-accommodating part (4) from its starting position relative to the central suture-release/suture-clamping part (5), swing open in a first direction such that the surface of the relevant front suture-accommodating part (4) is correspondingly increased in size in the direction of the central suture-release/suture-clamping part (5), and which, with guidance of the suture-accommodating part (4) back into its abovementioned starting position, can be moved back into their swung-in state again.

## Revendications

1. Dispositif de guidage d'au moins deux fils de suture (14, 15 ; 24, 25) à travers une paroi d'une enveloppe, d'un ballon ou d'une surface, notamment d'une artère d'un sujet, à proximité du bord d'une ouverture pratiquée dedans, éventuellement formée par incision et/ou découpe libre, et de retrait des extrémités du fil de suture guidées à travers la paroi concernée hors de l'ouverture mentionnée, comportant un dispositif de guidage de fil en forme de tige (1) dans lequel les fils de suture (14, 15 ; 24, 25) fixés à des aiguilles (12, 13 ; 26, 27) sont guidés dans des ouvertures de guidage ou d'admission (8, 9 ; 22, 23) de manière à pouvoir être guidés à travers la paroi de l'enveloppe, du ballon ou de la surface grâce au dispositif de guidage du fil concerné (1) et ressortis de l'ouverture mentionnée de manière à ce qu'en tirant ensemble les extrémités du fil de suture hors de l'ouverture concernée, celle-ci puisse être fermée,
**caractérisé en ce que**
le dispositif de guidage de fil (1) comporte, dans son sens longitudinal, une pièce arrière d'acheminement de fil (3), une pièce avant d'admission de fil (4) et une pièce centrale de libération/blocage de fil (5) située entre elles et que ladite pièce centrale de libération/blocage de fil (5) peut tourner du moins par rapport à la pièce avant d'admission de fil (4) et présente une section transversale telle qu'elle permette, dans au moins une position de rotation, l'introduction des fils de suture (14, 15 ; 24, 25) acheminés par la pièce arrière d'acheminement de fil (3) dans les ouvertures d'admission (22, 23) libres dans la pièce avant d'admission de fil (4) et permette de maintenir, dans une position de rotation différente de la position de rotation mentionnée, les fils de suture (14, 15 ; 24, 25) reçus avec les aiguilles (12, 13 ; 26, 27) dans les ouvertures d'admission concernées (22, 23) pour tirer l'ensemble du dispositif de guidage de fil (1) hors de l'ouverture mentionnée.

2. Dispositif selon la revendication 1, **caractérisé en ce que** la pièce arrière d'acheminement de fil (3), la pièce centrale de libération/blocage de fil (5) et la pièce avant d'admission de fil (4) présentent respectivement une section transversale de forme ovale.

3. Dispositif selon la revendication 2, **caractérisé en ce que** la pièce arrière d'acheminement de fil (3), la pièce centrale de libération/blocage de fil (5) et la pièce avant d'admission de fil (4) du dispositif d'acheminement de fil (1) présentent au moins dans leurs zones contiguës respectivement la même section transversale de forme ovale.

4. Dispositif selon la revendication 2, **caractérisé en ce que** la section transversale de la pièce centrale de libération/blocage de fil (5) est réduite au niveau de la largeur de sa section transversale par rapport aux sections transversales de la pièce arrière d'acheminement de fil (3) et de la pièce avant d'admission de fil (4).

5. Dispositif selon une des revendications 1 à 4, **caractérisé en ce que** la pièce arrière d'acheminement de fil (3), la pièce centrale de libération/blocage de fil (5) et la pièce avant d'admission de fil (4) du dispositif de guidage de fil (1) peuvent toutes tourner les unes par rapport aux autres.

6. Dispositif selon la revendication 1, **caractérisé en ce que** la pièce centrale de libération/blocage de fil (5) est formée par une zone de section transversale réduite de la pièce arrière d'acheminement de fil (3).

7. Dispositif selon la revendication 6, **caractérisé en ce que** la zone de section transversale réduite de la pièce arrière d'acheminement de fil (3) est formée par une pièce distincte (5), qui est reliée fixement avec la pièce arrière d'acheminement de fil (3).

8. Dispositif selon une des revendications 1 à 7, **caractérisé en ce que** la pièce centrale de libération/blocage de fil (5) présente une épaisseur (L2) correspondant à l'épaisseur de la paroi d'une enveloppe, d'un ballon ou d'une surface, notamment d'une artère d'un sujet.

9. Dispositif selon une des revendications 1 à 8, **caractérisé en ce que** dans la pièce arrière d'acheminement de fil (3) et la pièce avant d'admission de fil (4) m groupes, où m est ≥ 2, de n trous oblongs et d'admission (8, 9 ; 22, 23) adjacents, où n est ≥ 1, sont prévus.

10. Dispositif selon une des revendications 1 à 9, **caractérisé en ce que** la pièce arrière d'acheminement de fil (3) présente des trous oblongs (8, 9), dans lesquels les fils de suture (14, 15 ; 24, 25) fixés aux aiguilles (12, 13 ; 26, 27) peuvent être déplacés au moyen de tirettes séparées (18, 19) et introduits par l'intermédiaire de la pièce centrale de libération/blocage de fil (5) dans les trous d'admission (22, 23) alignés avec les trous oblongs (8, 9) de la pièce avant d'admission de fil (4) se trouvant dans sa position mentionnée.

11. Dispositif selon la revendication 10, **caractérisé en ce que** les trous d'admission (22, 23) de la pièce avant d'admission de fil (4) présentent une profondeur (L3) telle qu'au moins les aiguilles (12, 13 ; 26, 27) se trouvant aux extrémités avant des fils de suture (14, 15 ; 24, 25) peuvent être reçues intégralement dans ces trous d'admission (22, 23).

12. Dispositif selon la revendication 10 ou 11,
**caractérisé en ce que** les trous d'admission (22, 23) situés dans la zone avant de la pièce avant d'admission de fil (4) s'étendent en angle par rapport à leur axe longitudinal aligné avec l'axe longitudinal des trous oblongs (8, 9) de la pièce arrière d'acheminement de fil (3).

13. Dispositif selon une des revendications 10 à 12, **caractérisé en ce que** les ouvertures de guidage (8, 9) se trouvent au bord de la pièce d'acheminement de fil (3) et sont recouvertes par une pièce en film (10, 11) fixée à cette pièce d'acheminement de fil (3).

14. Dispositif selon la revendication 13, **caractérisé an ce que** les ouvertures de guidage (8, 9) pratiquées dans la pièce arrière d'acheminement de fil (3) sont placées à une distance telle de sa périphérie extérieure qu'une partie de la périphérie extérieure des aiguilles (12, 13 ; 26, 27) se trouve à l'extérieur de la périphérie extérieure de la pièce arrière d'acheminement de fil (3).

15. Dispositif selon une des revendications 10 à 14, **caractérisé en ce que,** dans la pièce d'acheminement de fil (3), à proximité des ouvertures de guidage (8, 9), se trouvent des chambres de réserve (28, 29) dans lesquelles sont logées des aiguilles supplémentaires (26, 27) reliées avec d'autres fils de suture (24, 25), qui arrivent, après l'introduction des aiguilles (12, 13) d'abord présentes dans les ouvertures de guidage mentionnées (8, 9) dans les ouvertures d'admission (22, 23) pratiquées dans la pièce avant d'admission de fil (4) et le retrait consécutif des tirettes (18, 19) avancées pour cette introduction, dans une position de retrait libérant les chambres de réserve concernées (28, 29), dans les ouvertures de guidage mentionnées (8, 9), dans lesquelles elles peuvent être introduites au moyen des tirettes mentionnées (18, 19) dans des ouvertures d'admission (22, 23) pratiquées dans la pièce avant d'admission de fil (4).

16. Dispositif selon la revendication 13, **caractérisé en ce que** servent pour l'admission des aiguilles supplémentaires mentionnées (26, 27) les mêmes ouvertures d'admission (22, 23) dans lesquelles sont reçues les aiguilles (12, 13) qui se sont d'abord trouvées dans les ouvertures de guidage (8, 9).

17. Dispositif selon la revendication 14 ou 15, **caractérisé en ce que** les aiguilles supplémentaires (26, 27) logées dans les chambres de réserve (28, 29) peuvent être introduites par force de ressort dans les ouvertures de guidage mentionnées (8, 9).

18. Dispositif selon une des revendications 14 à 17, **caractérisé en ce qu'**en cas de présence de trous d'admission supplémentaires dans la pièce avant d'admission de fil (4) avant l'introduction des aiguilles supplémentaires (26, 27) logées dans les chambres de réserve (28, 29) dans les trous d'admission supplémentaires mentionnés de la pièce avant d'admission de fil (4), celle-ci subit une torsion relative par rapport à la pièce arrière d'acheminement de fil (3) telle que les trous d'admission supplémentaires pratiqués dans la pièce avant d'admission de fil (4) sont alignés par rapport aux ouvertures de guidage (8, 9) pratiquées dans la pièce arrière d'acheminement de fil (3).

19. Dispositif selon une des revendications 15 à 18, **caractérisé en ce que** la pièce arrière d'acheminement de fil (3) et la pièce avant d'admission de fil (4) subissent, avant l'introduction des aiguilles supplémentaires mentionnées (26, 27) dans les ouvertures d'admission (22, 23) de la pièce avant d'admission de fil (4), une torsion relative par rapport à la pièce centrale de libération/blocage de fil (5).

20. Dispositif selon une des revendications 1 à 19, **caractérisé en ce que** la pièce centrale de libération/blocage du fil (5) est reliée, au moyen d'une pièce à manchon (7) traversant de manière à pouvoir tourner la pièce arrière d'acheminement de fil (3), avec une poignée (34) et que la pièce arrière d'acheminement de fil (3) et la pièce avant d'admission de fil (4) sont reliées, éventuellement par l'intermédiaire d'un dispositif à manchon (6) disposé coaxialement par rapport à la pièce à manchon (7) mentionnée, avec des roues de réglage de rotation (35, 36).

21. Dispositif selon la revendication 20, **caractérisé en ce que** les roues de réglage de rotation (35, 36) sont reliées avec des cliquets d'enclenchement (38, 40) qui permettent de régler les roues de réglage de rotation concernées (35, 36) et donc la pièce arrière d'acheminement de fil (3) ou la pièce avant d'admission de fil (4) reliée avec celles-ci dans des positions en angles définies par rapport à la poignée (34) et ainsi par rapport à la pièce centrale de libération/blocage de fil (5).

22. Dispositif selon la revendication 20 ou 21, **caractérisé en ce que** les roues de réglage de rotation (35, 36) pour la pièce arrière d'acheminement de fil (3) et pour la pièce avant d'admission de fil (4) sont couplées avec un mécanisme de verrouillage/libération (120 à 123) de manière à ce que le déplacement des aiguilles (12, 13 ; 26, 27) par les tirettes respectives (18, 19 ; 114) soit lancé simplement lorsque la pièce d'acheminement du fil (3) et la pièce d'admission du fil (4) sont alignées l'une par rapport à l'autre.

23. Dispositif selon la revendication 22, **caractérisé en ce que** le déplacement des aiguilles (12, 13 ; 26, 27) est lancé dans le cas où la pièce centrale de libération/blocage de fil (5) se trouve dans sa position de libération du fil.

24. Dispositif selon une des revendications 1 à 23, **caractérisé en ce que** la pièce arrière d'acheminement de fil (3) et la pièce avant d'admission de fil (4) sont traversées par un élément de guidage (100) à l'aide duquel la pièce avant d'admission de fil (4) peut être tournée par rapport à la pièce arrière d'acheminement de fil (3).

25. Dispositif selon la revendication 24, **caractérisé en ce que** l'élément de guidage (100) est constitué par un fil de guidage (100).

26. Dispositif selon la revendication 24 ou 25, **caractérisé en ce que** la pièce arrière d'acheminement de fil (3) est traversée par une pièce à manchon (101) entourant l'élément de guidage (100), grâce à laquelle la pièce centrale de libération/blocage de fil (5) peut tourner par rapport à la pièce arrière d'acheminement de fil (3) .

27. Dispositif selon une des revendications 1 à 26, **caractérisé en ce que** la pièce avant d'admission de fil (4) présente, sur sa surface tournée vers la pièce centrale de libération/blocage de fil (5), des éléments à ressort (60) relevables et rabattables qui, sur torsion de la pièce avant d'admission de fil (4) à partir de sa position initiale par rapport à la pièce centrale de libération/blocage de fil (5), se relèvent dans une première direction de manière à ce que la surface de la pièce avant d'admission de fil concernée (4) soit agrandie en conséquence dans la direction de la pièce centrale de libération/blocage de fil (5), et qui peuvent être ramenés dans leur position rabattue sur renvoi de la pièce d'admission de fil (4) vers sa position initiale mentionnée.
